# EUROPEAN PATENT SPECIFICATION

(11) **EP 1 469 791 B1**
(45) Date of publication and mention of the grant of the patent: **06.02.2008**
(21) Application number: 02789974.9
(22) Date of filing: 03.12.2002
(51) Int. Cl.: A61F 2/06

(54) **MULTI-SEGMENT MODULAR STENT AND METHODS FOR MANUFACTURING STENTS**
AUS MEHREREN SEGMENTEN BESTEHENDER MODULARER STENT UND VERFAHREN ZUR HERSTELLUNG VON STENTS
ENDOPROTHESE MODULAIRE A SEGMENTS MULTIPLES ET PROCEDES DE FABRICATION D'ENDOPROTHESES

(30) Priority: 03.12.2001 US 337060 P
(43) Date of publication of application: 27.10.2004
(73) Proprietor: Intek Technology LLC, Wilmington, DE 19808 (US)
(72) Inventor: RABKIN, Dimitry, J., Chestnut Hill, MA 02467 (US); MORAG, Eyal, East Hampton, MA 01027 (US); PERELSON, Ophir, Beverly Hills, CA 90211 (US)
(74) Representative: Molnia, David
(86) International application number: PCT/US2002/038456
(87) International publication number: WO 2003/047464

(56) References cited:
- EP-A- 0 873 728
- WO-A-01/32102
- WO-A-97/25937
- CA-A1- 2 272 667
- DE-U- 29 615 969
- US-A- 5 681 346
- US-A- 5 800 508
- US-A- 5 938 697
- US-A- 6 042 597
- US-A1- 2001 047 201
- US-B1- 6 270 524
- US-B1- 6 517 573
- US-B2- 6 503 271
- US-B2- 6 514 063

## Description

### Field of the Invention

This invention relates generally to medical devices, and more particularly to radially expandable stents for holding vessels such as arteries for open flow, and to methods for manufacturing stents.

### Background of the Invention

A stent is a generally longitudal cylindrical device formed of biocompatible material, such as metal or plastic, which is used in the treatment of stenosis, strictures, or aneurysms in body blood vessels and other tubular body structures, such as the esophagus, bile ducts, urinary tract, intestines or the tracheo-bronchial tree.

A stent is held in a reduced diameter unexpanded configuration within a low profile catheter until delivered to the desired location in the tubular structure, most commonly a blood vessel, whereupon the stent radially expands to an expanded diameter configuration in the larger diameter vessel to hold the vessel open. Radial expansion may be accomplished by an inflatable balloon attached to a catheter, or the stent may be of the self-expanding type that will radially expand once released from the end portion of the delivery catheter. A fundamental concern is that the stent be as completely apposed to the vessel wall as possible, exerting maximal focal radial forces at the site of the narrowing.

Generally, there are several desired objectives in designing a stent. One objective is to provide the stent with an optimal distribution of radial forces along its length in its expanded configuration so that the stent provides a uniform, high radial force in the stenosed region of the vessel but a lower radial force in healthy parts of the vessel where high forces are not necessary. A stent should be able to counteract two main extrinsic forces, namely the elastic recoil of the atherosclerotic plaque and the adjacent non-diseased vessel wall, and active contraction of smooth muscle fiber within the vessel wall. In addition, the stent should be maximally apposed to the vessel wall to minimize the relative motion between the vessel wall and the struts from which the stent is constructed, which may result in intimal trauma. The stent should exert enough focal radial force to open the narrowed segment. However, the remaining vessel segments do not necessarily need to be exposed to these stretching forces.

Another objective in stent design is to provide the stent with a high degree of flexibility in its unexpanded or collapsed configuration in order to facilitate maneuvering within tortuous vessels during delivery, as well as optimum flexibility of the stent in its expanded configuration for better wall apposition when deployed within tortuous vessels. It has been demonstrated experimentally that better apposition of the stent struts to the vessel wall is associated with improved long-term patency of the stented vessel. A stent which is not completely apposed to the vessel wall results in more exuberant intimal response and a higher incidence of restenosis. Poor stent apposition in a pulsating artery may be associated with repetitive micro trauma to the vessel wall, again resulting in an increase in the incidence of clot formation and restenosis.

The apposition to the vessel wall should be balanced with the "metal to wall" ratio, meaning that the healthy vessel should be exposed to the least surface area of the metallic stent. At the same time the diseased segment should be exposed to the minimum force required to open it wide, while preventing the plaque from extending and protruding through the stent struts.

Another criteria of stent design is to provide a flexible stent which is also kink resistant in order to decrease overlapping of stent struts and the protrusion of exposed edges of the struts of a curved stent into the wall of a tortuous vessel.

Stents in actual use today are generally uniform in their design and for the most part are constructed from interconnected struts forming either a plurality of identical interconnected annular Z-rings or a plurality of identical interconnected annular closed-cell rings. Each type of ring possesses the main inherent feature of radial expansion following deployment. The closed-cell rings can incorporate different cell designs, which are intended to provide better radial forces and wall apposition.

Multi-segment stents, i.e. stents having a non-uniform design including both Z-rings and closed-cell rings, have been described in the prior art and are designed as such for different purposes. Examples of such stent designs are shown in U.S. Pat. No. 5064435 to Porter; U.S. Pat. No. 5354308 to Simon; U.S. Pat. No.5569295 to Lam; U.S. Pat. No.5716393 to Lindenberg; U.S. Pat. No. 5746765 to Kleshinski; U.S. Pat. No.5807404 to Richter et al; U.S. Pat. No.5836966 to St. Germain; U.S. Pat. No.5938697 to Killion; U.S. Pat. No. 6146403 to St. Germain; U.S. Pat. No.6159238 to Killion; U.S. Pat. No.6187034 to Frantzen; U.S. Pat. No.6231598 to Berry et al.; U.S. Pat. No.6106548 to Roubin et al.; U.S. Pat. No.6066168 to Lau et al.; U.S. Pat. No.6325825 to Kula et al.; U.S. Pat. No.6348065 to Brown et al.; U.S. Pat. No.6355057 to DeMarais et al and U.S. Pat. No. 6355059 to Richter et al.

US 59 38 697 discloses a stent comprising an intermediate closed cell segment and a pair of end Z-segments.

Some of these designs attempt to address problems which are encountered in clinical practice including inadequate wall apposition, overlapping of neighboring struts and incomplete cell expansion leading to insufficient radial force distribution. Some of them are constructed to provide variable radial forces while some are designed to be flexible to maintain good wall apposition. However, the stents described in the prior art generally are specifically designed to provide only one or two of these features and therefore only meet a limited number of the desired objectives.

Stents are typically manufactured from thin tubes which are slotted by a laser beam to define a series of closely-packed struts. However, this technique has certain problems and limitations. One problem is in the manufacture of self expanding stents which are not uniform in design, e.g. multi-segment stents. Such stents are typically manufactured from thin tubes of shape memory alloy which are slotted by a laser beam to define a plurality of interconnected closed-cell rings and Z-rings, and then mechanically expanding the tubes on mandrels to progressively greater diameters and at the same time heat treating them to impart the desired temperature-shape memory characteristics. However, as a non-uniform multi-segment stent is mechanically expanded, the struts forming the annular rings are subjected to assymmetrical forces resulting in irregular or distorted closed-cell and Z-ring geometry. This irregular geometry is "memorized" by the stent so that upon delivery to and expansion in a stenosed region of a vessel, it will not provide optimal force distribution or wall apposition.

Another problem arises in the manufacture of stents from a laser slotted tube when it is desired that the tube wall be very thin so that the struts formed from the slotted tube wall are correspondingly thin, such as when the stent is to be expanded in a small diameter vessel. In order to prevent the thin tube material at the vertices of intersecting struts from tearing as the tube is expanded during manufacture, it has been necessary for the slots formed by the laser beam to be a certain, relatively large, width to provide a large radius curvature at the vertices of the struts to relieve the stresses in those regions as the tube expands. However, this limits the width of the struts.

Still another problem in the manufacture of non-uniform multi-segment stents comprising a plurality of interconnected closed-cell rings and Z-rings by laser-cutting and then expanding small diameter tubes is that it is often costly and time consuming to create specific software for guiding the laser cutting tool to cut the particular desired sequence and configuration of closed-cell rings and Z-rings. The need to create specific laser cutting tool software for a particular predetermined desired sequence and arrangement of closed-cell rings and Z-rings for a stent has impeded the widespread adoption and use of multi-segment stents having annular rings sequenced and arranged to provide optimal characteristics for a particular clinical application.

### Summary of the Invention

It is therefore an object of the present invention to provide a new and improved stent designed to provide optimal features for a wide range of clinical applications.

Another object of the present invention is to provide a new and improved stent designed to provide optimal radial forces, flexibility and kink resistance for a wide range of clinical applications.

Still another object of the present invention is to provide a new and improved stent designed to provide optimal radial forces, flexibility and kink resistance taking into account specific anatomic locations of the lesion or stenosis and the geometry and other characteristics of the lesion or stenosis.

A further object of the present invention is to provide a new and improved stent designed to optimally distribute radial forces along its length.

A still further object of the present invention is to provide a new and improved stent with a high degree of flexibility in its unexpanded configuration for maneuvering within tortuous vessels during delivery.

Yet another object of the present invention is to provide a new and improved stent with optimal flexibility in its expanded and deployed condition for improved wall apposition in tortuous vessels.

A still further object of the present invention is to provide a new and improved flexible stent which is kink resistant to decrease the exposure of sharp edges of the struts of the stents in tortuous vessels.

Briefly, these and other objects are attained by providing a stent having a modular construction constituted by at least two modules, one of the modules including an intermediate segment consisting of closed-cell segment, and a pair of end segments connected by interconnector elements to respective axial ends of said intermediate segment, each end segment consisting of Z-segment. Each Z-segment consists solely of at least one annular Z-ring formed by an elongate member shaped or constructed to include a plurality of generally sinusoidal or waveshape portions defining proximal and distal peaks and valleys. Each closed-cell segment consists solely of at least one annular ring formed by a pair of longitudinally adjacent Z-rings which are tightly interconnected to each other to form a ring of circumferentially interconnected closed-cell elements defining proximal and distal peaks and valleys. In a "Type A" module, the intermediate segment comprises a Z-segment and each of the pair of end segments comprises a closed-cell segment. In a "Type B" module, the intermediate segment comprises a closed-cell segment and each of the pair of end segments comprises a Z-segment. The stent comprises at least a type B module and one of either a type A or a type B module.

Preferably, the stents are formed of modules in which each closed-cell segment of a module comprises from one to four closed-cell rings and each Z-segment of a module comprises from one to eight Z-rings.

Each Z-ring and each closed-cell ring preferably defines from four to sixteen distal and proximal peaks and valleys. Longitudinally adjacent pairs of rings are interconnected by interconnector elements connected to opposed or offset pairs of peaks and/or valleys of the connected rings, and in the case of adjacent closed-cell rings, by shared walls or struts of the closed-cells.

Stents formed of at least 2 modules having the aforesaid construction will possess three desirable characteristics namely, a distribution of radial force along the length of the stent appropriate for any particular case, a high degree of kink resistance and a high degree of longitudinal flexibility (in both unexpanded and expanded configurations) thereby making such a stent suitable for a wide range of applications.

For example, a stent in accordance with the invention can provide strong radial forces along a stenosed portion of a vessel which shows the largest burden of atherosclerotic plaque by covering this area with closed-cell segments, which have higher radial force and stability. Depending on the length of these lesions closed-cell segments can be constructed of one or more rings to cover the entire area of the stenosis.

A multi-segment modular stent having this construction is also particularly suited for use in portions of vessels having sharp turns. In such cases, a closed-cell segment is preferably situated at the apex of a sharp turn in the vessel to prevent kinking of the stent and maintain good patency and flow through the stent. This construction also eliminates exposure of free edges of stent struts at the bend. An area of significant narrowing at the apex of a curvature of a vessel should be covered with a closed cell segment of the modular stent to prevent kinking, as well as for providing greater radial support. The adjacent Z-segments will allow the device to conform to the angles and tortuous geometry of the vessel.

Tortuous portions of a vessel without any significant narrowing are covered with Z-ring segments. In the case of an S-shaped vessel configuration with a mild disease along its entire length, it is beneficial to place a long segment of Z-rings to cover this area. Z-segments should also be positioned at the turns of a vessel between significant tandem lesions, which should be covered with closed-cell segments.

In cases of relatively straight vessels it is beneficial to use modular stents with closed-cell segments at both ends for better anchoring and stability. On the other hand if portions of a vessel immediately adjacent to an area of significant narrowing are tortuous or have bends, it would be better to use a modular stent with Z-ring segments at the ends for better apposition to the vessel wall.

The multi-segment modular construction also provides stability to the stent to minimize frictional motion resulting from vessel pulsation. This motion is believed to contribute to constant microtrauma and aseptic inflammatory changes within the vessel wall, which in turn results in formation of excessive neointima which grows through the stent struts causing eventually restenosis.

A long modular stent constructed according to the invention can be used in patients who otherwise require placement of more than one standard stent. This technique will avoid both the undesirable overlap of stents, which often leads to higher incidence of more prominent intimal hyperplasia and restenosis, and the potential for leaving uncovered gaps between stents, which can lead to protrusion of an atherosclerotic plaque and flow compromise. Long modular stents can be built to accommodate complex vessel shapes which are difficult or impossible to cover with sequential placement of several standard stents.

A stent of the present invention may be constructed from a shape memory alloy such as nitinol for self-expanding stents or from stainless steel or other alloys for balloon-expandable stents. The self-expanding stent expands spontaneously as a result of superelasticity combined with the shape memory effect of exposure to body temperature and in several designs presented herein, is designed to exhibit minimal or no foreshortening.

In order to manufacture multi-segment self-expanding stents having a particular predetermined desired sequence and arrangement of closed-cell rings and Z-rings, with the rings all having a regular, undistorted geometry, in accordance with the invention, the small diameter tube is laser-cut to define a plurality of longitudinally adjacent Z-rings interconnected by interconnector portions so that every pair of adjacent Z-rings constitutes a closed-cell ring. The tube is expanded and heat-treated, and then certain ones of the interconnector portions are removed from the expanded tube to provide the predetermined desired sequence and arrangement of interconnected closed-cell rings and Z-rings. All of the interconnector portions, including the interconnector portions which are eventually removed, serve to maintain the regular geometry of the rings during expansion and heat treatment of the tube.

Finally, in order to facilitate the manufacture and use of multi-segment stents, stent blanks are initially prepared, which may be done even before the desired sequence and arrangement of the Z-rings and closed-cell rings have been determined. A blank is formed by laser-cutting a small diameter tube of shape-memory material to define a plurality of pairs of longitudinally adjacent Z-rings having interconnector portions integrally joining the Z-rings of each pair in a manner such that every pair of adjacent Z-rings constitutes a closed-cell ring. The small diameter tube is then expanded and heat treated to form a stent blank. Once the particular intended application of the stent is known, the particular desired sequence and arrangement of the interconnected closed-cell rings and Z-rings are determined. Certain ones of the interconnector portions are then removed from the blank, either mechanically or using a laser tool, in order to provide the desired arrangement and sequence of the closed-cell rings and Z-rings. This technique enables an inventory of blanks for multi-segment stents to be maintained so that once a particular clinical application is determined for a stent, it is a simple and quick matter to obtain an appropriate stent blank and remove appropriate interconnector portions to provide the stent with optimal features for the particular application.

### Brief Description of the Drawings

A more complete appreciation of the present invention and many of the attendant advantages thereof will be readily understood by reference to the following detailed description when considered in connection with the accompanying drawings in which:
Fig. 1 (a) shows one embodiment of a Z-ring in its expanded configuration, cut longitudinally and flattened into a plane;
Fig. 1(b) is similar to Fig. 1(a) and shows another embodiment of a Z-ring;
Figs. 2(a)-2(1) show different embodiments of closed-cell rings in expanded configurations, cut longitudinally and flattened into a plane;
Fig. 3 shows the angle between two struts forming a peak of a Z-ring, or a peak of a closed-cell ring, in an expanded configuration;
Fig. 4 shows the distance d between the distal and proximate peaks of two longitudinally adjacent annular rings in an expanded configuration;
Figs. 5(a)-5(g) show pairs of longitudinally adjacent Z-rings interconnected by different embodiments of interconnectors in expanded configurations, cut longitudinally and flattened;
Fig. 6 shows a hexagonal type closed-cell ring formed of a pair of longitudinally adjacent Z-rings interconnected to each other by interconnectors at every pair of opposed peaks, in an expanded configuration, cut longitudinally and flattened;
Fig. 7 shows a pair of longitudinally adjacent closed-cell rings interconnected by linear interconnector elements, in an expanded configuration, cut longitudinally and flattened;
Figs. 8(a)-8(c) show pairs of longitudinally adjacent closed-cell rings interconnected by interconnector elements in the form of shared walls, in expanded configurations, cut longitudinally and flattened;
Fig. 9 shows an embodiment of a Type A stent module invention in an expanded configuration, cut longitudinally and flattened;
Fig. 10 shows an embodiment of a Type B stent module invention in an expanded configuration, cut longitudinally and flattened;
Fig. 11 shows another embodiment of a Type A stent module in an expanded configuration, cut longitudinally and flattened;
Fig. 12 shows another embodiment of a Type B stent module in an expanded configuration, cut longitudinally and flattened;
Fig. 13 shows two Type A stent modules interconnected by shared walls of closed-cell elements of adjacent annular closed-cell rings, in an expanded configuration, cut longitudinally and flattened;
Fig. 14 shows the two Type A stent modules of Fig. 13, but interconnected by linear interconnectors connecting opposed peaks of adjacent closed-cell rings, in an expanded configuration, cut longitudinally and flattened;
Fig. 15 shows an embodiment of a stent in accordance with the present invention formed of an intermediate Type B module and a pair of Type A modules interconnected to the ends of the Type B module, in an expanded configuration, cut longitudinally and flattened;
Fig. 16 is a front elevation view of another embodiment of a stent in accordance with the present invention formed of an intermediate Type B module and a pair of Type A modules interconnected to the ends of the Type B module;
Fig. 17 is a front elevation view of the stent shown in Fig. 16, shown in its expanded configuration and bent about 180°;
Fig. 18 is a front elevation view of the stent shown in Figs. 16-17, shown in its expanded configuration and situated within an S-shaped tortuous vessel;
Fig. 19 shows a laser-slotted tube constituting the stent shown in Figs. 16-18 in an unexpanded configuration, cut longitudinally and flattened;
Fig. 20 shows another embodiment of a Type A stent module adapted for placement across a side branched vessel, in an expanded configuration, cut longitudinally and flattened;
Fig. 21 is a schematic perspective view showing a hollow fenestrated wire for forming a stent through which fluids, such as medication, can be delivered;
Fig. 22 shows another embodiment of a stent in accordance with the present invention formed of an intermediate Type B module and a pair of Type B modules interconnected to the ends of the intermediate Type B module, in an expanded configuration, cut longitudinally and flattened;
Fig. 23 shows another embodiment of a stent in accordance with the present invention formed of an intermediate Type B module and a pair of Type A modules interconnected to the ends of the intermediate Type B module, in an expanded configuration, cut longitudinally and flattened;
Fig. 24(a) shows a portion of a laser-slotted tube, cut longitudinally and flattened;
Fig. 24(b) shows a step of a progressive mechanical expansion of the laser-slotted tube shown in Fig. 24(a), cut longitudinally and flattened;
Fig. 25(a) shows a portion of a laser-slotted tube used in stent manufacture, cut longitudinally and flattened;
Fig. 25(b) shows a magnified area, designated B, of the laser-slotted tube shown in Fig. 25(a);
Fig. 25(c) shows a step of a progressive mechanical expansion of the laser-slotted tube shown in Figs. 25(a) and 25(b) during manufacture;
Fig. 25(d) shows a stent manufactured from the expanded laser-slotted tube shown in Figs. 25(a)-25(c), cut longitudinally and flattened;
Fig. 25(e) is a view similar to Fig. 25(b) showing another example of a laser-slotted tube used in stent manufacture;

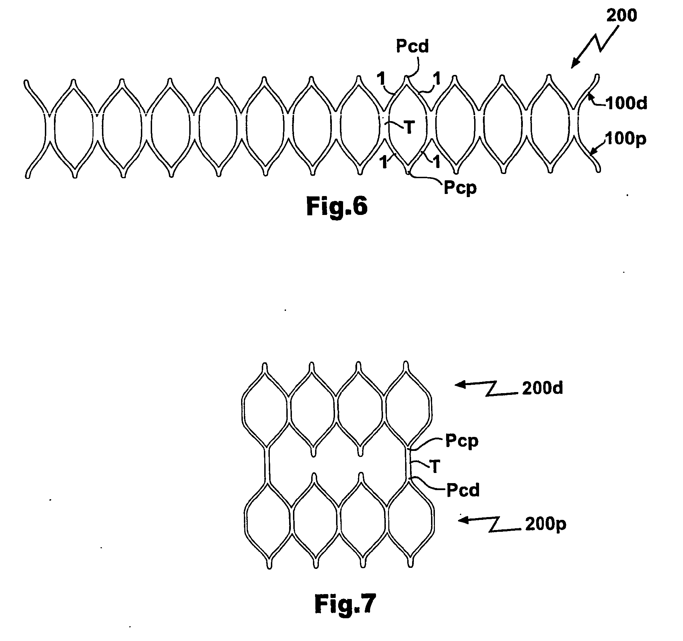

Fig. 6 is a front elevation view of a tapered stent in accordance with the present invention formed of an intermediate Type B module and a pair of Type A modules interconnected to the ends of the intermediate Type B module;

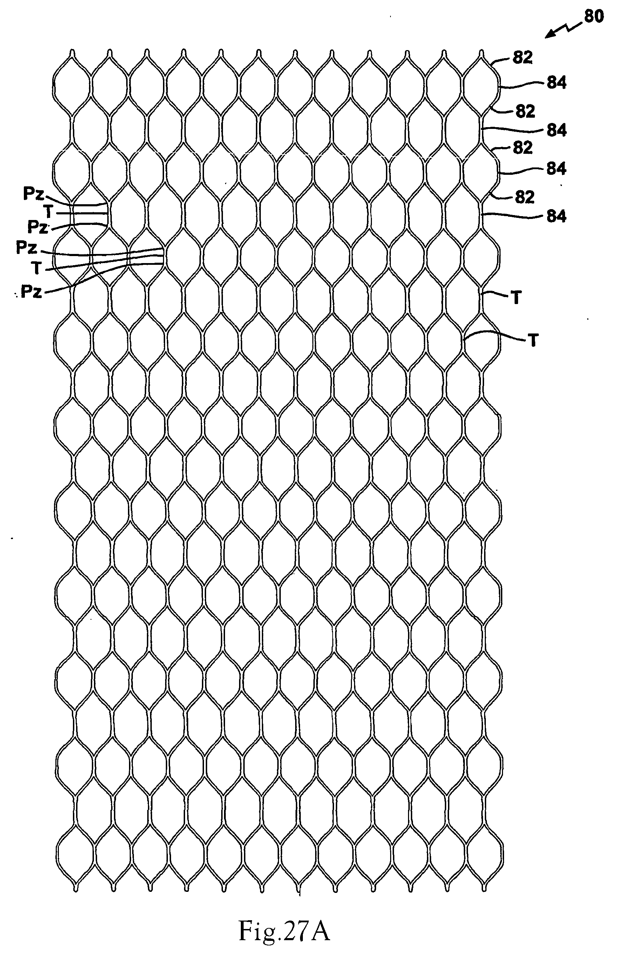

Fig. 27(a) shows a first example of a stent blank, cut longitudinally and flattened;

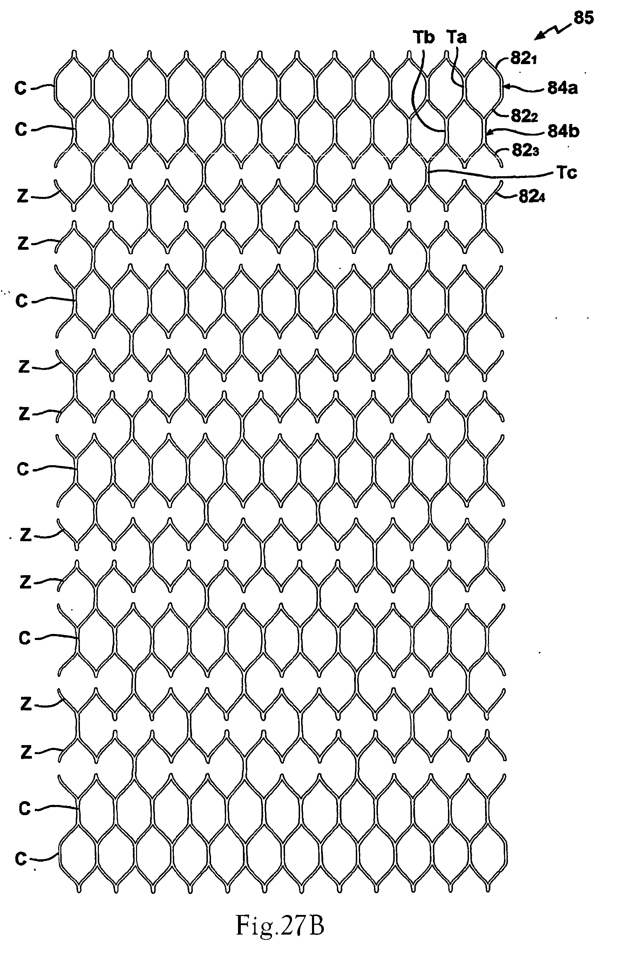

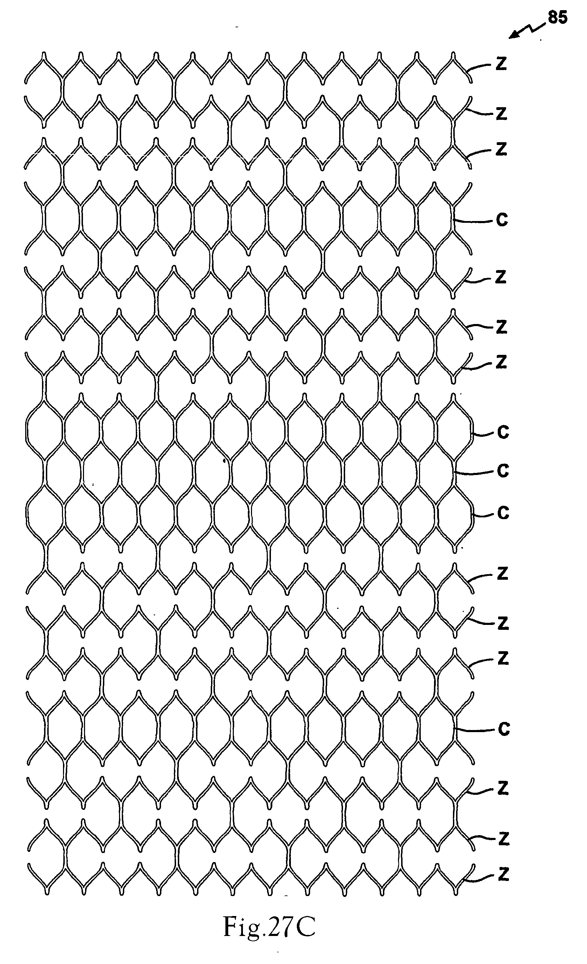

Figs. 27(b) and 27(c) show different stents manufactured from the stent blank of Fig. 27(a), cut longitudinally and flattened;

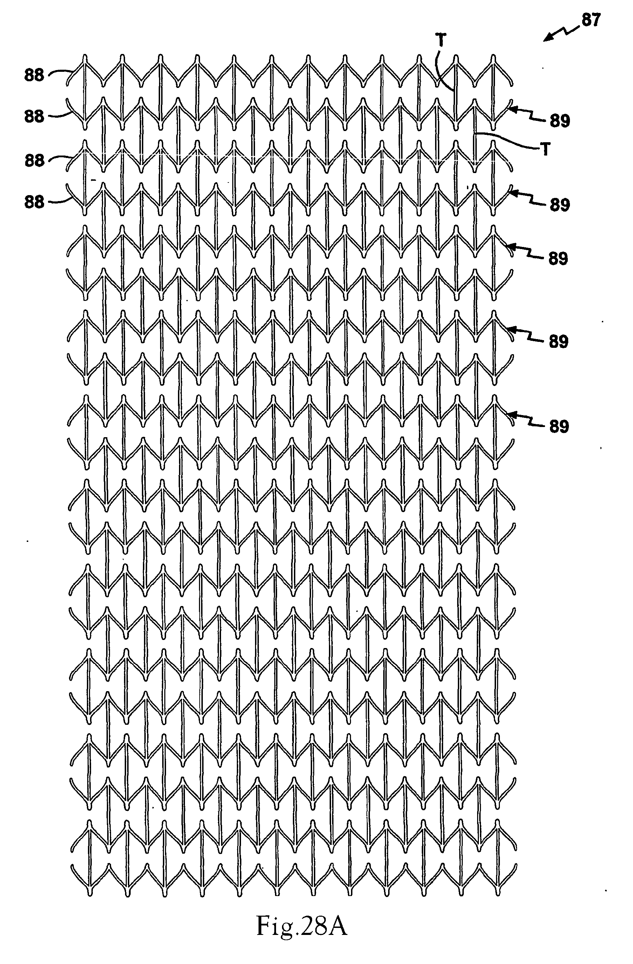

Fig. 28(a) shows a second example of a stent blank cut longitudinally and flattened;

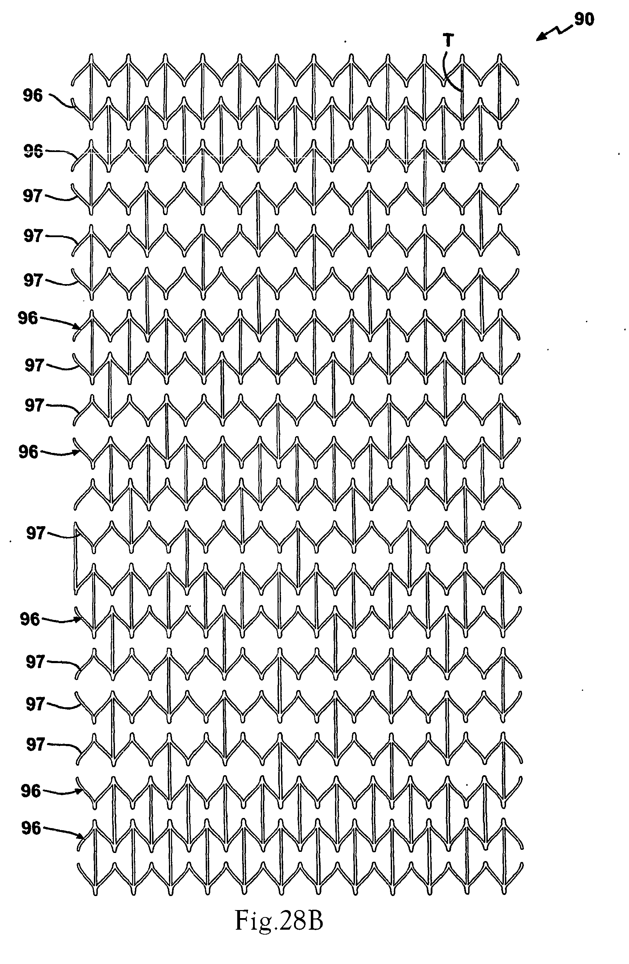

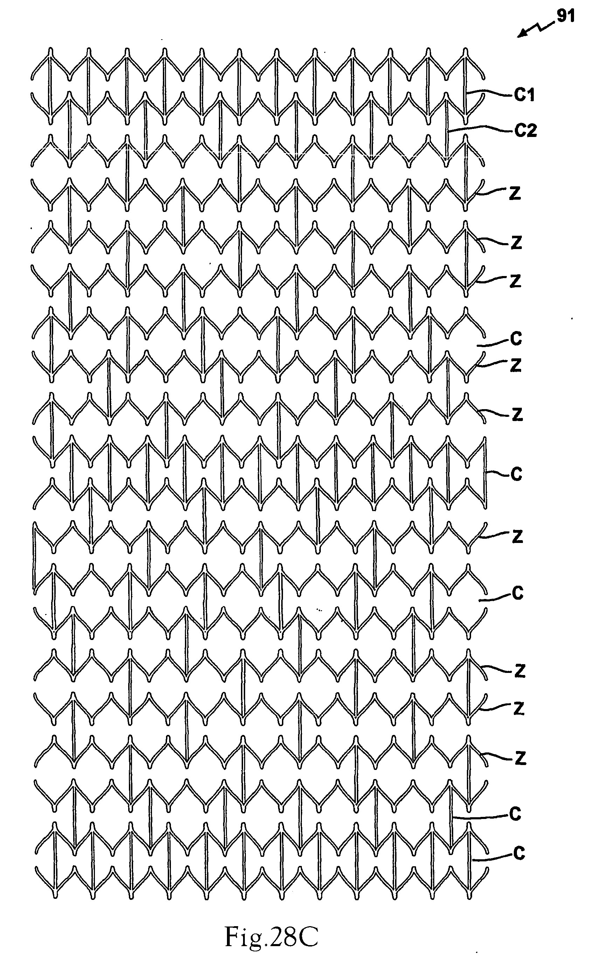

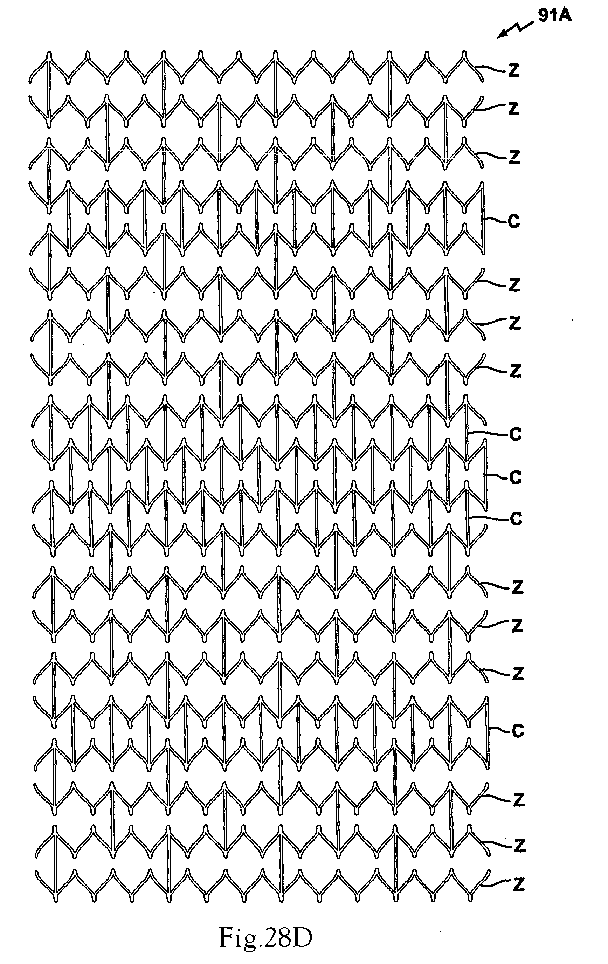

Figs. 28(b), 28(c) and 28(d) show different stents manufactured from the stent blank of Fig. 27(a), cut longitudinally and flattened;

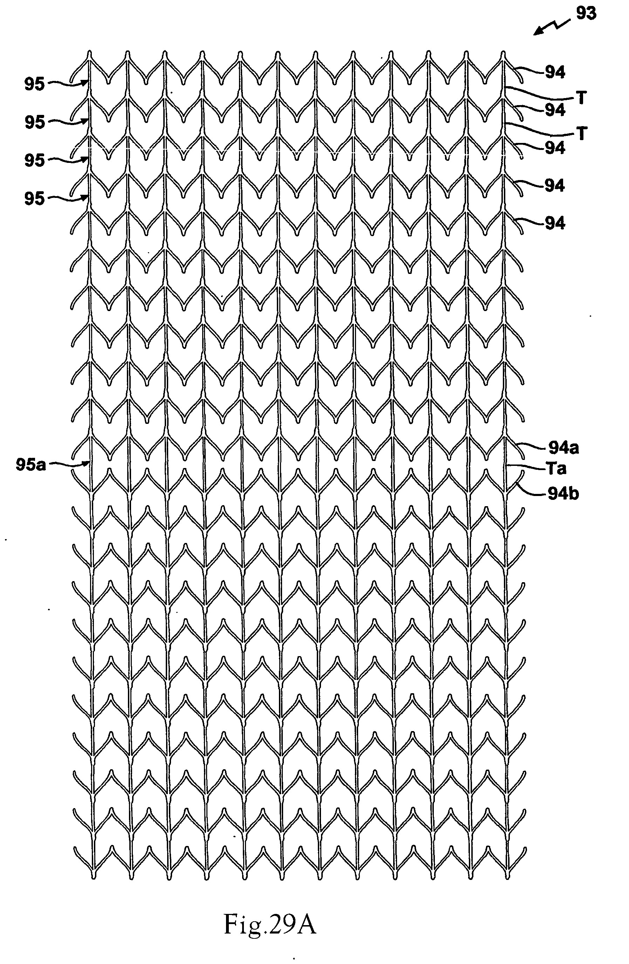

Fig. 29(a) shows a third example of a stent blank, cut longitudinally and flattened; and

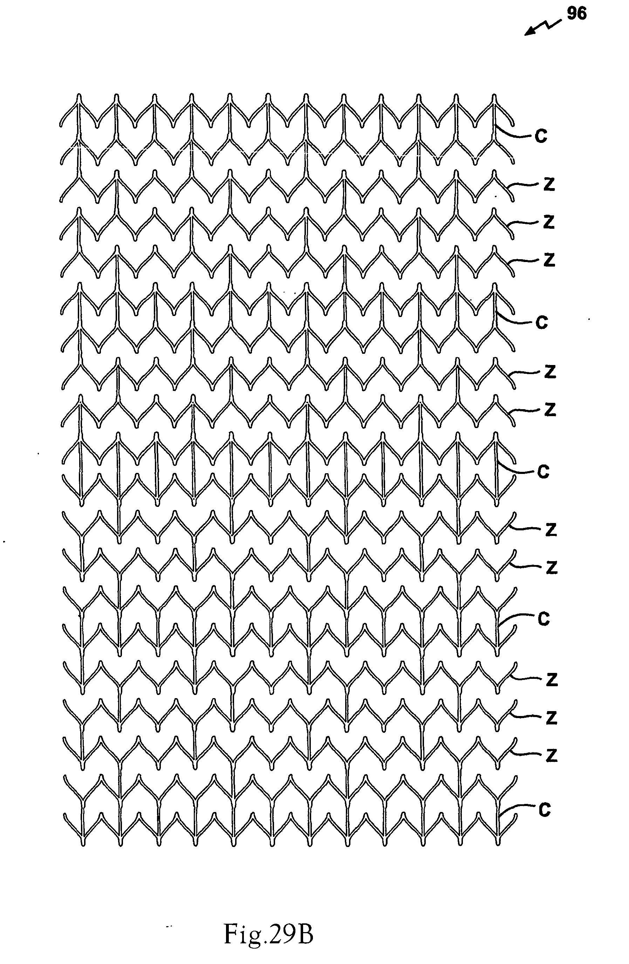

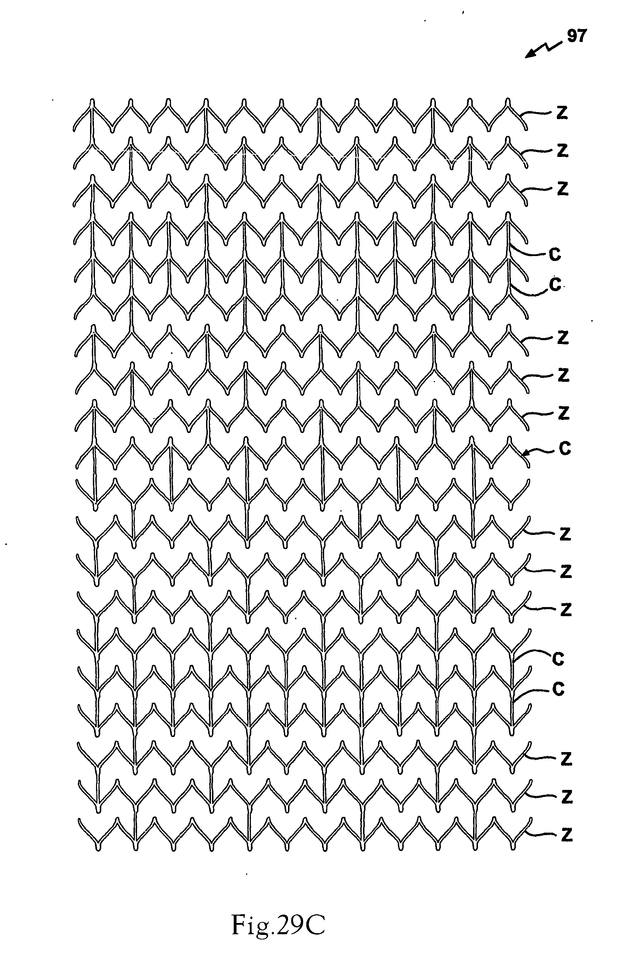

Figs. 29(b) and 29(c) show different stents manufactured from the stent blank of Fig. 29(a), cut longitudinally and flattened.

### Description of the Preferred Embodiments

A stent in accordance with the invention has a modular construction constituted by a combination of interconnected segments of annular Z-rings and closed-cell rings. Each module is formed of three segments including an intermediate segment comprising either a closed-cell segment or a Z-segment and a pair of end segments comprising the other of closed-cell or Z-segments.

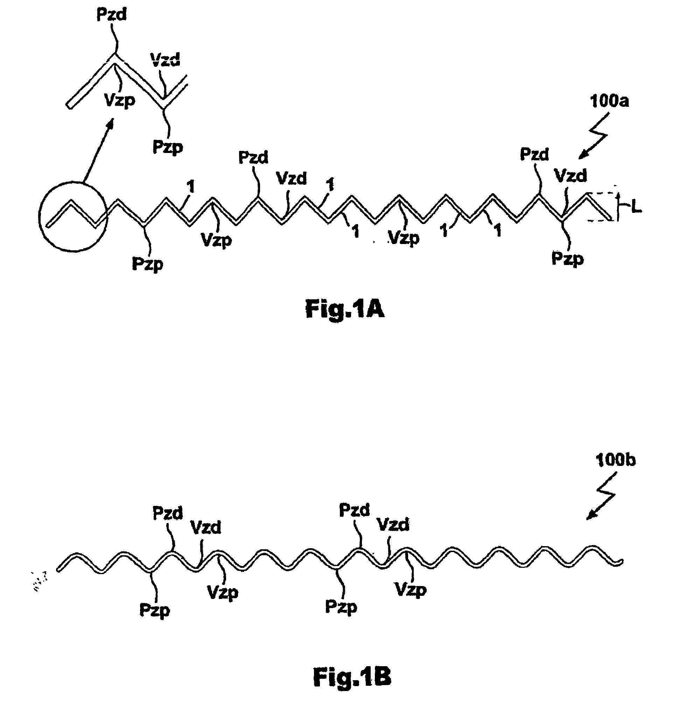

Referring now to the drawings wherein like reference characters designate identical or corresponding parts throughout the several views, and more particularity to Fig. 1(a), a Z- ring 100a comprises struts 1 which together define a plurality of "Z" or sinusoidal or wave shapes. The struts 1 may be formed by expanding a laser-slotted metallic tube, or from portions of a single wire, or from individual wire elements, or by any other method of construction known to those skilled in the art. The mesh design of the stent can be laser cut from a large diameter tube, which is equal to the final diameter of a fully expanded stent or which may be further expanded to an even larger diameter. This technological process enables the steps of expansion and heat treatment steps to be avoided and enables perfectly uniform shape of the cells throughout the stent to be achieved. However larger tubes are more expensive and there is substantial amount of wasted material during the laser cutting process.

The Z-ring 100a has twelve distal peaks P(z)_{d} and twelve proximal peaks P(z)ₚ constituted by the most distal and most proximal longitudinal edge surfaces of the ring 100a. The distal longitudinal direction is designated in this and other drawing figures by arrow L, i.e. upward toward the top of the page. In this case, the peaks P(z)_{d}, P(z)ₚ are the outermost edge surfaces of the vertices of pairs of intersecting struts 1. Twelve distal and proximal valleys V(z)_{d} and V(z)ₚ are constituted by the innermost edge surfaces associated with each peak on the distal and proximal sides of the Z-ring, i.e. facing in the distal and proximal directions. In this case the valleys V(z)_{d}, V(z)ₚ are at the inner sides of the vertices of pairs of intersecting struts 1.

While the struts of the Z-ring 100a shown in Fig. 1 (a) are integrally formed with each other and intersect each other at sharp points, the Z-rings can have other forms. For example, referring to Fig. 1(b), an annular Z-ring 100b comprises twelve integral smooth sinusoidal waves having peaks P(z)_{d}, P(z)ₚ and valleys V(z)_{d}, V(z)ₚ. Z-rings forming Z-segments of a stent module in accordance with the invention preferably comprise between four and sixteen distal and proximal peaks P(z)_{d} and P(z)ₚ and valleys V(Z)_{d} and V(z)ₚ over their circumference.

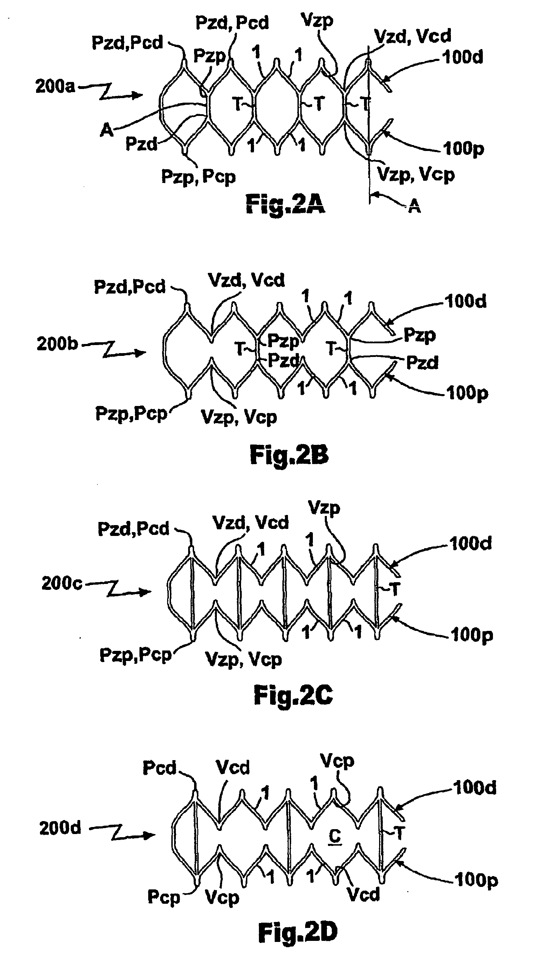

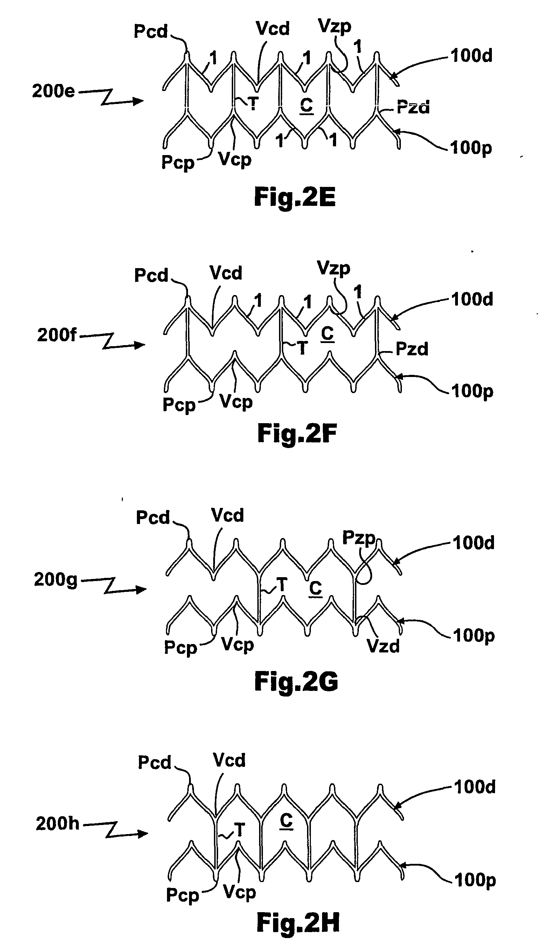

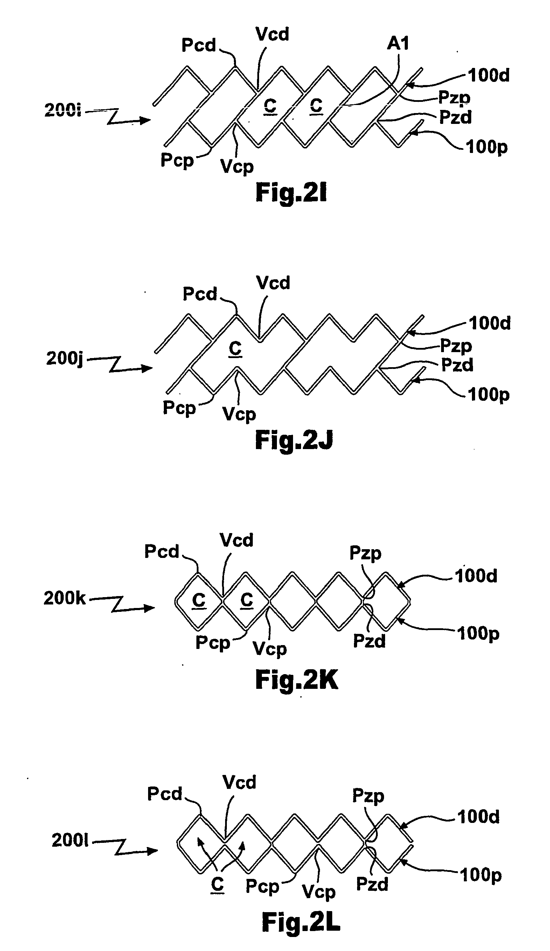

Referring to Fig. 2, closed-cell rings are shown formed by pairs of tightly interconnected longitudinally adjacent Z-rings. The Z-rings can either be longitudinally aligned, i.e. mutually positioned with their pairs of proximate peaks (and their proximate valleys) P(z)_{P} and P(z)_{d} in longitudinal alignment, indicated by the longitudinally extending line A in Fig. 2(a) which passes through proximate aligned peaks, or longitudinally offset, i.e. mutually positioned with their proximate peaks P(z)ₚ and P(z)_{d} being offset from each other, indicated by the oblique line A₁ in Fig. 2(i), which passes through proximate offset peaks P(z)ₚ and P(z)_{d}. In this case (e.g. Fig. 2(i)), each peak in one Z-ring is longitudinally aligned with a respective proximate valley in the other Z-ring as indicated by the line A₂ in Fig. 2(i). By "tightly interconnected" is meant that the Z-rings are connected to each other at every, or at every other, pair of proximate aligned or proximate offset peaks or valleys or their combination.

As seen in Fig. 2(a), a closed-cell ring 200a having a plurality of interconnected hexagonal-shaped closed-cell elements C, is formed by interconnecting two aligned Z-rings 100_{d} and 100ₚ at every pair of facing proximal and distal peaks P(z)ₚ, P(z)_{d} of distal and proximal Z-rings 100_{d} and 100ₚ, by means of straight, longitudinally extending interconnectors T. A closed-cell ring of this type has a high radial strength, a high degree of kink resistance, low flexibility and a relatively high degree of foreshortening upon expansion from its collapsed state.

Referring to Fig. 2(b), a closed-cell ring 200b having a plurality of interconnected closed-cell elements C is formed by interconnecting two aligned Z-rings 100_{d} and 100ₚ at every other pair of facing peaks P(z)ₚ and P(z)_{d} with linear and longitudinal interconnectors T. This type of closed-cell ring will provide less radial strength, less kink resistance, somewhat more flexibility and the same degree of foreshortening as the closed-cell ring 200a shown in Fig. 2(a). Closed-cell rings of this type have large open areas and therefore provide a smaller metal to vessel wall ratio, which may be beneficial in certain clinical situations, but at the same time can allow prolapse of atherosclerotic material through the interstices.

A closed-cell annular ring 200c, shown in Fig. 2(c), having a plurality of closed-cell elements C, is formed by interconnecting every proximal valley V(z)ₚ of a distal Z-ring 100_{d} with every facing distal valley V(z)_{d} of an aligned proximal Z-ring 100_{P} by a linear longitudinal interconnector T. This type of closed-cell ring is comparable in flexibility and kink resistance to the one shown in Fig. 2(a). It has somewhat less radial strength, but has a benefit of less foreshortening upon expansion as compared to the design shown in Fig. 2(a). Referring to Fig. 2(d), a closed-cell ring 200d having a plurality of closed-cell elements C is shown which is similar to ring 200c of Fig. 2(c), with the exception that every other pair of opposed valleys V(z)ₚ and V(z)_{d} are interconnected, thereby increasing the area enclosed by each individual closed-cell, and reducing the overall metal to vessel wall ratio. It has slightly less radial force and kink resistance, slightly more flexibility and the same degree of foreshortening as the design shown on Fig. 2(c). Referring to Fig. 2(e), a closed-cell annular ring 200e having a plurality of closed-cell elements C is formed by interconnecting every proximal valley V(z)ₚ of a distal Z-ring 100_{d} with the facing distal peak P(z)_{d} of an offset proximal Z-ring 100ₚ by linear longitudinal interconnectors T. The annular ring 200e has less radial strength, similar flexibility and kink resistance, but a slightly less degree of foreshortening as compared to the design shown in Fig. 2(a). It has more prominent foreshortening compared to the design shown in Fig. 2(c). Referring to Fig. 2(f), closed-cell ring 200f is shown formed of a plurality of closed-cell elements C having a construction similar to that shown in Fig. 2(e), except that every other pair of facing proximal valleys V(z)ₚ and distal peaks P(z)_{d} of offset distal and proximal Z-rings 100_{d} and 100ₚ are interconnected, resulting in larger open areas for the individual closed cell elements C and a smaller overall metal to vessel wall ratio. Referring to Fig. 2(g), a closed-cell ring 200g is formed including a plurality of closed-cell elements C by interconnecting every other distal valley of the V(z)_{d} of proximal Z-ring 100ₚ with every other aligned proximal peak of the offset distal Z-ring 100_{d} by linear longitudinal interconnectors T. This closed-cell ring 200g has identical features to the closed-cell ring 200(f) shown in Fig. 2(f). Referring to Fig. 2(h), a closed-cell ring 200h formed of closed-cell elements C is formed by interconnecting every pair of aligned proximal peaks P(z)ₚ and distal valleys V(z)_{d} of offset distal and proximal Z-rings 100_{d} and 100ₚ with linear longitudinal interconnectors T. The closed-cell ring 200h has identical features to the ring 200e shown in Fig. 2(e). Referring to Fig. 2(i), a closed-cell annular ring 200i is formed by a pair of offset Z-rings 100_{d} and 100ₚ by interconnecting every distal peak P(Z)_{d} of proximal Z-ring 100ₚ with every facing proximal peak P(z)ₚ of offset Z-ring 100_{d} with a linear, obliquely extending interconnector T. Closed-cell ring 200i includes a plurality of quadrilateral closed-cell elements C and has a higher degree of stability than the closed-cell rings described above. Referring to Fig. 2(j), a closed-cell annular ring 200j including closed-cell elements C is formed by a pair of offset proximal and distal Z-rings 100ₚ and 100ₐ by interconnecting every other distal peak P(z)_{d} of proximal Z-ring 100_{P} with a facing proximal peak P(z)ₚ of distal Z-ring 100_{d}. A closed-cell ring 200j provides a higher degree of flexibility and less radial strength compared to the closed-cell ring 200i of Fig. 2(i). As shown in Fig. 2(k), a closed-cell ring 200k is formed by directly, i.e without linear interconnectors, connecting every pair of opposed peaks P(z)ₚ, and P(z)_{d} of aligned distal and proximal Z-rings 100_{d} and 100_{P}. As shown in Fig. 2(l), a closed-cell ring 2001 can also be formed by directly connecting every other pair of opposed peaks P(z)ₚ, and P(z)_{d} of adjacent aligned Z-rings 100_{d} and 100_{P}.

Each of the closed-cell rings 200 shown in Fig. 2 has its own proximal and distal peaks P(c)ₚ and P(c)_{d} and proximal and distal valleys V(c)p and V(c)_{d}. The proximal peaks P(c)ₚ and proximal valleys V(c)ₚ of each closed-cell ring 200 correspond to the proximal peaks P(z)ₚ and proximal valleys V(z)ₚ of the proximal Z-ring 100_{P} forming part of the closed-cell ring 200 while the distal peaks P(c)_{d} and distal valleys V(c)_{d} of each closed-cell ring 200 correspond to the distal peaks P(z)_{d} and distal valleys V(z)_{d} of the distal Z-ring 100ₐ forming part the closed-cell ring 200. Closed-cell rings forming closed-cell segments of a stent module in accordance with the invention preferably comprise between four and sixteen distal and proximal peaks P(c)_{d} and P(c)ₚ and valleys V(c)_{d} and V(c)ₚ over their circumference.

Stents in accordance are constructed of both closed-cell rings and Z-rings in a particular modular arrangement to achieve an optimal combination of several main characteristics, including appropriate radial force distribution in the axial direction, good longitudinal flexibility, in both expanded and unexpanded configurations, good kink resistance, and reduced foreshortening upon expansion. Longitudinal stent portions that comprise closed-cell segments generally exhibit greater radial force, and lesser flexibility, i.e. greater stiffness, than stent portions formed of Z-segments. Struts forming closed-cell rings and closed-cell segments have greater geometric stability than struts forming Z-rings and Z-segments. For this reason, stent portions comprising closed-cell segments exhibit less relative motion between the stent struts and the vessel wall than do stent portions formed of Z-segments. Increased stability of the stent struts with consequent decrease in relative movement between the stent and the apposed vessel wall results in a reduced potential for inflammation of the vessel wall.

Compared to stent portions formed of Z-segments, stent portions formed of closed-cell segments exhibit increased kink resistance and therefore improved integrity of the inner lumen of the stent. Stents portions formed of Z-segments generally tend to kink to a greater extent even in vessels having relatively small bends. Kinking of stent portions formed of Z-segments results in overlapping of, and interference between the struts which protrude into the stent lumen in regions of curvature, thereby reducing flow through the stent lumen. The walls of relatively long curved vessels will not be well supported by Z-segments due to separation of the stent struts at the greater curvature of the vessel.

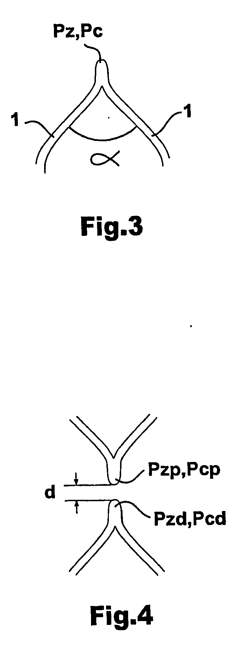

The longitudinal flexibility of a stent, the radial force distribution over the length of a stent, and the resistance to kinking of a stent, are all also influenced by the geometry of the closed-cell and Z-rings themselves. Referring to Fig. 3, the radial forces exerted by an expanded stent portion formed of either closed-cell segments or Z-segments increase as the angle between two intersecting struts 1 defining a peak P(c) or P(z) of a closed-cell ring or Z-ring of the fully expanded stent increases. On the other hand, as the angle increases, it becomes more difficult to collapse the stent into its unexpanded configuration after manufacture in preparation for preloading and delivery of the stent using a catheter system. The angle α is preferably in the range of between 35° to 65°, depending on the diameter of the stent in its expanded configuration, the desired radial force, the number of peaks in each annular ring, and the thickness of the struts.

Referring to Fig. 4, the distance d between longitudinally adjacent distal and proximate peaks P(z)_{d}, P(c)_{d;} P(z)ₚ, P(c)ₚ of two adjacent annular rings also affects both flexibility and kink resistance of a stent portion which includes those annular rings. Specifically, increasing the distance d will increase the longitudinal flexibility of the stent while decreasing the stent's resistance to kinking. The distance d should be sufficiently great to prevent any significant overlapping of adjacent struts when the stent is bent and, at the same time, small enough to provide the necessary vessel wall support in large curvature portions.

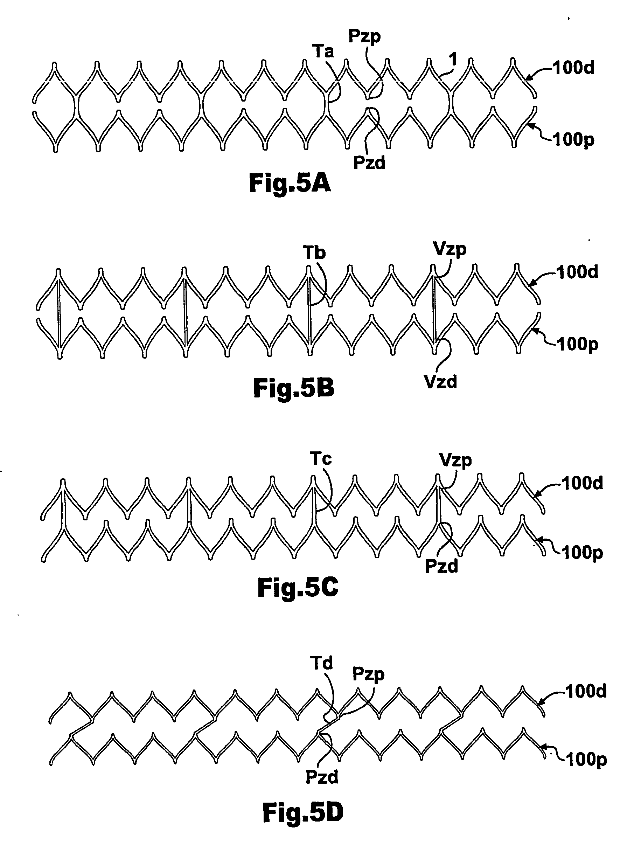

The shape, length, width and spacing of the interconnectors interconnecting longitudinally adjacent Z-rings to form a Z-segment, as well as interconnecting longitudinally adjacent Z-rings and closed-cell rings, discussed below, all affect the flexibility, kink resistance and radial force of stent portions including those interconnected rings. Moreover, the degree to which the stent foreshortens upon expansion from its unexpanded to its expanded configuration is also affected by the geometric characteristics of the interconnectors. Referring to Fig. 5(a), adjacent distal and proximal Z-rings 100_{d} and 100ₚ are longitudinally aligned and the distal peaks P(z)_{d} of the proximal Z-ring 100_{P} are situated in aligned facing relationship to the proximal peaks P(z)ₚ of the distal Z-ring 100_{d}. Longitudinally extending linear interconnectors Tₐ, having a width equal to or somewhat greater than the width of the struts 1, interconnect every third pair of aligned facing peaks P(z)_{d}. P(z)ₚ to form a two-ring Z-segment. Generally, increasing the length of the interconnectors Tₐ increases the flexibility of the segment, decreases the radial force provided by the segment and decreases the kink resistance of the segment. Generally, increasing the width of the interconnectors Tₐ decreases the flexibility of the stent portion, increases the kink resistance of the stent portion and does not materially affect the radial force of the stent portion, compared to thinner interconnectors of the same length. Increasing the spacing between circumferentially adjacent interconnectors Tₐ, such as to every fourth pair of aligned peaks from every third shown in Fig. 5(a), generally increases the flexibility of the stent portion but decreases the kink resistance, while the radial force remains about the same.

Referring to Fig. 5(b), longitudinally aligned proximal and distal Z-rings 100_{P} and 100_{d} are interconnected to form a Z-segment by longitudinal linear interconnectors T_{b} which interconnect every third pair of aligned valleys V(z)_{d}, and V(z)ₚ. Stent portions incorporating Z- rings interconnected in this manner exhibit less foreshortening upon expansion than stent portions incorporating Z-rings interconnected as shown in Fig. 5(a). The other characteristics of the stent portions remain about the same.

Referring to Fig. 5(c), longitudinally offset proximal and distal Z-rings 100ₚ and 100ₐ are interconnected by linear, longitudinal interconnectors T_{c} that interconnect every third pair of opposed peaks and valleys P(z)_{d}, and V(z)ₚ of Z-rings 100ₚ and 100_{d}. A stent portion incorporating annular rings interconnected in the manner of Fig. 5(c) has good flexibility and improved support and coverage of vessel walls at areas of curvature. It will also exhibit decreased foreshortening on expansion.

Referring to Fig. 5(d), the longitudinally offset proximal and distal Z- rings 100ₚ, 100_{d} are interconnected by linear interconnectors T_{d} that extend obliquely between every third distal peak P(z)_{d} of proximal Z-ring 100ₚ and an offset facing proximal peak P(z)ₚ of distal Z-ring 100_{d}. Stent potions incorporating Z- rings of this type exhibit greater flexibility than, for example, stent portions incorporating Z-rings interconnected in the manner shown in Fig. 5(a).

Referring to Fig. 5(e), the longitudinally aligned proximal and distal Z- rings 100ₚ and 100_{d} are interconnected by serpentine-shaped interconnectors Tₑ which interconnect every third pair of aligned peaks P(z)ₚ, and P(z)_{d} of distal and proximal Z-rings 100ₐ and 100ₚ. Stent portions incorporating Z-rings interconnected in this manner exhibit less foreshortening upon expansion than in the case of Fig. 5(a).

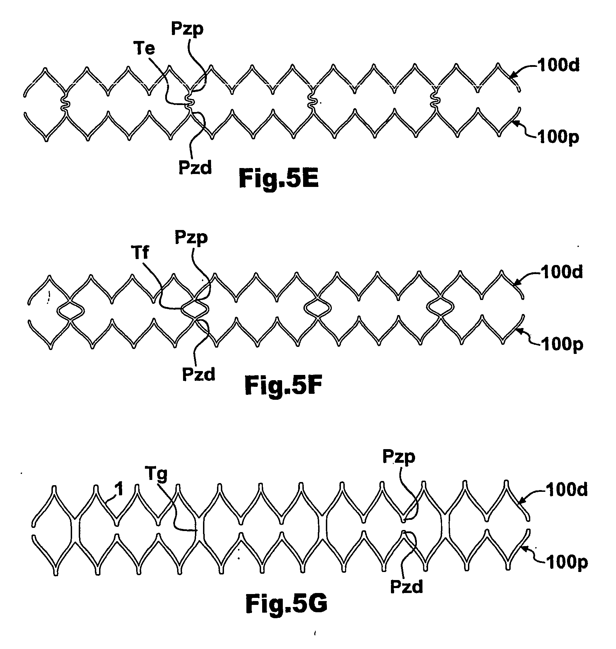

Referring to Fig. 5(f), two longitudinally aligned Z-rings 100_{d} and 100ₚ are interconnected by interconnectors T_{f}, which connect every third pair of aligned peaks P(z)_{d}, P(z)ₚ. Each interconnector T_{f} comprises a pair of outwardly bowed struts. Stent portions incorporating Z-rings interconnected in this manner exhibit a lesser degree of foreshortening than in the case of Fig. 5(a).

Referring to Fig. 5(g), two longitudinally aligned Z- rings 100ₚ and 100_{d} are interconnected by linear, longitudinal interconnectors T_{g} which interconnect every third pair of opposed peaks P(z)_{d}, and P(z)ₚ. However, unlike the construction of the interconnectors Tₐ of Fig. 5(a), each interconnector T_{g} has a width of about twice the width of the struts 1 forming the Z-rings. Increasing the thickness of the interconnectors T_{g} has the effect of simplifying the process for manufacturing stents including such interconnected Z-rings.

Referring to Fig. 6, a hexagonal-type closed-cell ring 200 is shown formed of a distal Z-ring 100_{d} interconnected to an aligned proximal Z-ring 100ₚ by interconnectors T having a width of about twice the thickness of the struts 1 defining the peaks P(c)ₚ, P(c)_{d}. The increased width of the interconnectors T serves to simplify the manufacturing process of stents incorporating closed-cell rings constructed in this manner.

The manner in which adjacent closed-cell rings are interconnected in closed-cell segments affects the characteristics of stent portions incorporating those segments in much the same way as the manner in which Z-rings are interconnected affects the characteristics of stent portions incorporating those segments. Referring to Fig. 7, two longitudinally aligned closed-cell rings 200_{d} and 200ₚ are interconnected by longitudinally extending linear interconnectors T which interconnect every third pair of aligned peaks P(c)ₚ, and P(c)_{d}. The configuration, length, width and spacing of the interconnectors T can be varied in a manner analogous to that discussed above in connection with the interconnection of Z-rings with generally similar effects on the properties of the stent portions incorporating those interconnected rings.

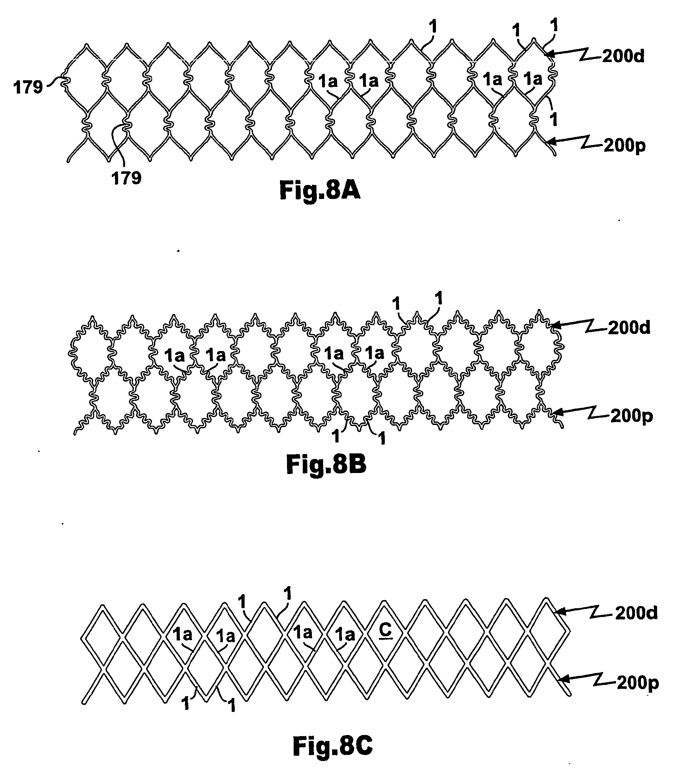

Referring to Figs. 8(a) through 8(c), examples of pairs of adjacent, longitudinally offset closed-cell annular rings 200ₐ and 200ₚ, interconnected by shared walls or struts 1a, are shown. In particular, Fig. 8(a) illustrates a pair of longitudinally offset proximal and distal closed-cell rings 200ₚ and 200_{d} having hexagonal type closed-cell elements C are interconnected by shared walls or struts 1a. The pairs of Z-rings forming each closed-cell ring are interconnected by wavy-shaped interconnectors 179. Interconnecting offset closed-cell rings 200_{d}, 200ₚ using shared walls 1a, rather than linear interconnectors, such as interconnectors T in Fig. 7, has the effect of increasing the radial force, increasing the kink resistance and decreasing the flexibility of a stent portion incorporating such interconnected closed-cell rings. Utilizing a wavy-shaped interconnector 179 has the effect of decreasing foreshortening upon expansion. Fig. 8(b) shows a pair of longitudinally offset closed-cell rings 200_{d} and 200_{P} interconnected by shared walls or struts 1a. All of the struts 1, 1a have a wavy configuration which increases the flexibility of a stent portion incorporating such interconnected closed-cell rings. Referring to Fig. 8(c), a pair of longitudinally adjacent offset closed-cell annular rings 200ₚ and 200_{d} is shown which are interconnected by shared walls 1a and in which each of the closed-cell elements C has a diamond shape, which makes the closed-cell segment somewhat shorter, compared to Figs. 8(a) and 8(b) and provides relative decrease in flexibility and increase in kink resistance.

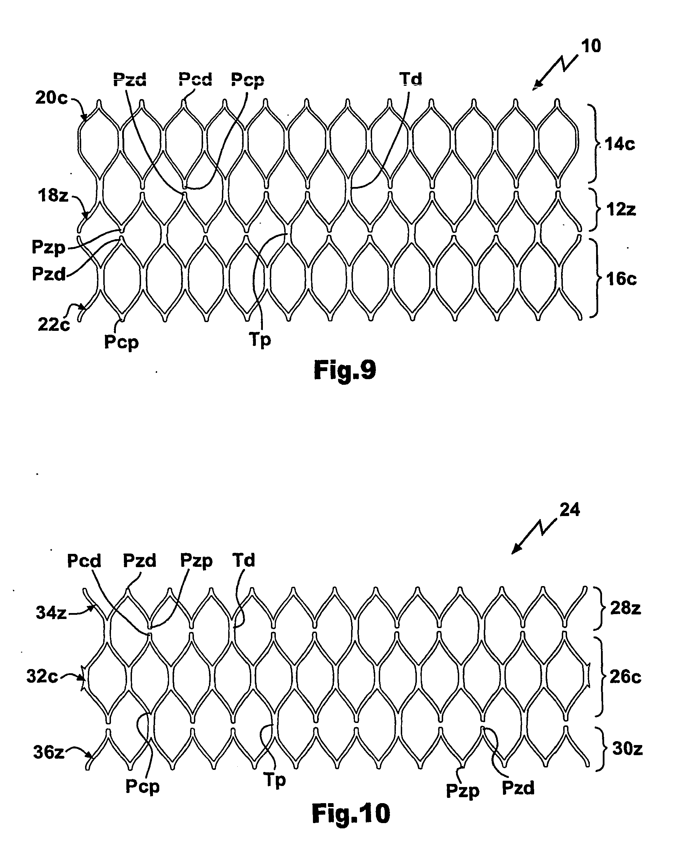

Referring to Fig. 9, a stent module 10 is shown in its expanded configuration, cut longitudinally and flattened. The module 10 can constitute an entire stent or can be interconnected to other modules so as to define an axial length portion of a modular stent incorporating several stent modules.

Stent module 10 is formed of an intermediate Z-segment 12z and distal and proximal closed-cell segments 14c and 16c interconnected to the axial ends of Z-segment 12z. A module of this type, i.e., including an intermediate Z-segment and a pair of closed-cell end segments, i.e., a "C-Z-C" type module, is designated a Type A module. The stent module 10 is the most simple in construction of Type A stent modules in that each of the three segments comprise only a single annular ring. Specifically, the intermediate Z-segment 12z is formed of a single annular Z-ring 18z having twelve distal peaks P(z)_{d} and twelve proximal peaks P(z)ₚ. The distal closed-cell segment 14c is formed of a single closed-cell annular ring 20c of the hexagonal type shown in Fig. 2(a) having twelve distal peaks P(c)_{d} and twelve proximal peaks P(c)ₚ. The proximal closed-cell segment 16c is formed of a single hexagonal-type closed-cell annular ring 22c having twelve distal peaks P(c)_{d} and twelve proximal peaks P(c)ₚ.

In stent module 10, the pairs of longitudinally adjacent annular rings, namely, rings 20c and 18z, and rings 18z and 22c, are longitudinally aligned with respect to each other, i.e. their opposed peaks P(c)_{d}, P(z)_{d}; P(z)ₚ; P(c)_{d} are longitudinally aligned with each other. Specifically, the twelve proximal peaks P(c)ₚ of closed-cell ring 20c are longitudinally aligned with the twelve distal peaks P(z)_{d} ofZ-ring 18z. Similarly the twelve distal peaks P(c)_{d} of closed-cell ring 22c are longitudinally aligned with the twelve proximal peaks P(z)ₚ of Z-ring 18z.

The intermediate Z-ring 18z is interconnected to each of the distal and proximal closed-cell rings 20c, 22c by four longitudinally extending linear interconnectors, T_{d} and Tₚ respectively, of the type shown in Fig. 5(a), interconnecting every third pair of opposed peaks of the pairs of adjacent annular rings. The interconnector elements Tₚ interconnecting the proximal side of the intermediate Z-ring 18z to the distal side of annular closed-cell ring 22c are situated circumferentially midway between each adjacent pair of interconnector elements T_{d} interconnecting the distal side of the intermediate Z-ring 18z to the proximal side of the distal annular closed-cell ring 20c. The uniform spacing of the interconnector elements facilitates a uniform expansion of a stent incorporating module 10.

A stent constructed from a plurality of interconnected modules 10 will provide a high degree of radial force along relatively long axial length portions, and relatively low flexibility and kink resistance along its length due to the repetition of closed-cell segments comprising pairs of longitudinally adjacent closed-cell rings (at the connected ends of adjacent modules) separated by Z-segments of only single Z-rings. Such a stent is useful in treating a relatively long stenosis in a relatively straight, or only somewhat curved, vessel. The use of linear interconnectors Tₚ and T_{d} in the manner shown and described provides the stent with some degree of flexibility and uniform expansion characteristics.

A stent constructed from a plurality of interconnected Type A modules in general will provide good radial force distribution over the length of the stent while the flexibility of the stent can be increased by adding additional Z-rings to Z-segments of the modules.

It will be understood that a module of the type shown in Fig. 9 can be constructed with closed-cell rings having configurations other than the type shown in Fig. 2(a), namely, with one of the closed-cell ring types shown in Figs. 2(b)-2(1), in which case the specific above-discussed features associated with such configuration will be obtained. Similarly, the manner of interconnection between adjacent rings can be of any of the types shown in Figs. 5(a)-5(g), 6, 7 and 8(a)-8(c), in which case the specific above-discussed features associated with such interconnectors will be obtained.

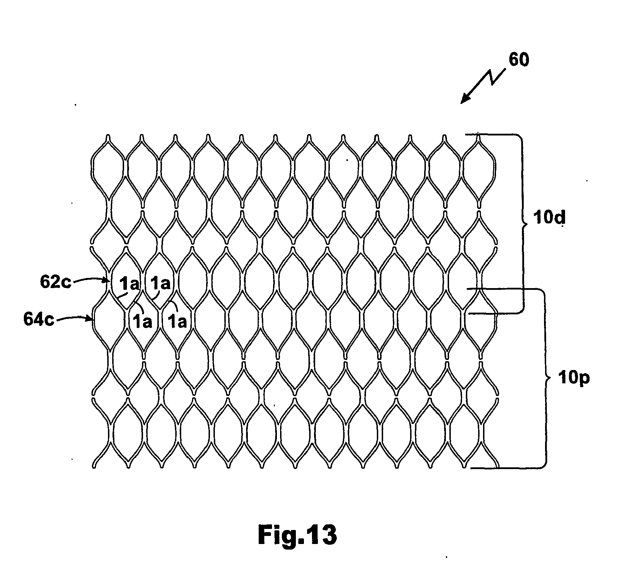

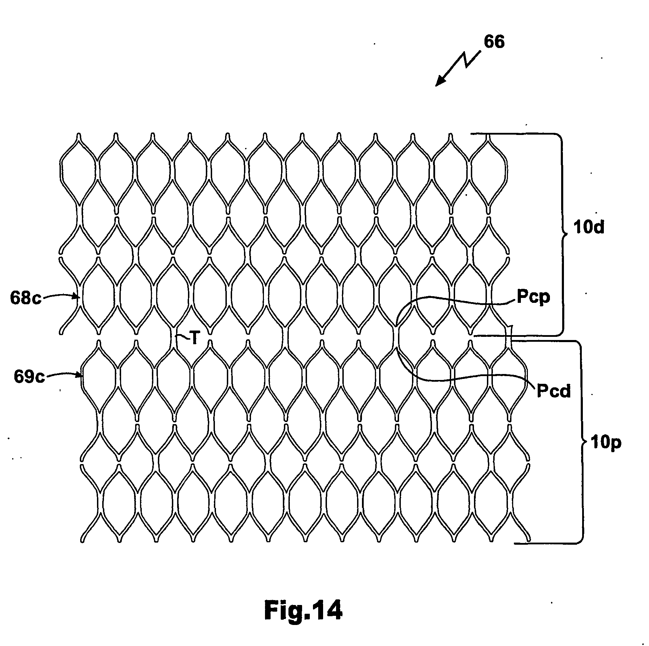

Moreover, the manner of interconnection between adjacent rings of two interconnected modules 10 can be varied. For example, referring to Fig. 13, an axial length portion 60 of a stent is illustrated formed of two interconnected Type A modules, 10_{d} and 10ₚ, each having the construction of module 10 shown in Fig. 9. In this embodiment the proximal closed-cell ring 62c of the distal module 10_{d} and the distal closed-cell ring 64c of proximal module 10ₚ are interconnected by shared walls or struts 1a of closed-cell rings 62c, 64c. Alternatively, referring to Fig. 14, another axial length portion 66 of a stent is illustrated formed of two Type A modules 10_{d} and 10ₚ, each having the construction of module 10 shown in Figs. 9 and 13. In this case, the proximal closed-cell ring 68c of the distal module 10_{d} and the distal closed-cell ring 69c of the proximal module 10ₚ are interconnected by means of four linear interconnectors T interconnecting opposed peaks P(c)ₚ and P(c)_{d} of rings 68c and 69c, respectively, spaced at every third pair of opposed peaks. The axial stent length portion 60 shown in Fig. 13 has less flexibility than the axial stent length portion 66 shown in Fig. 14 in view of the shared wall interconnection of the modules in the case of the former and the use of linear interconnectors T in the case of the latter, so that the axial stent length portion 66 shown in Fig. 14 is indicated for use where there is a sharper bend in the vessel, i.e. the vessel segment has a shorter radius of curvature.

Referring to Fig. 10, a stent module 24 is formed of an intermediate closed-cell segment 26c and distal and proximal Z-segments 28z and 30z interconnected to the axial ends of closed-cell segment 26c. A module of this type, i.e. including an intermediate closed-cell segment and a pair of end Z-segments, i.e., a "Z-C-Z" type module, is designated a Type B module.

The stent module 24 is the most simple in construction of Type B stent modules in that each of the three segments comprise only a single annular ring. Specifically, the intermediate closed-cell segment 26c is formed of a single annular closed-cell ring 32c of the hexagonal type shown in Fig. 2(a) having twelve distal peaks P(c)_{d} and twelve proximal peaks P(c)ₚ. The distal Z-segment 28z is formed of a single annular Z-ring 34z having twelve distal and proximal peaks P(z)_{d}, P(z)ₚ. The proximal Z-segment 30z is formed of a single annular Z-ring 36z having twelve distal peaks P(z)_{d} and twelve proximal peaks P(z)ₚ.

Like module 10 of Fig. 9, the longitudinally adjacent rings of module 24 are mutually positioned with their opposed peaks in longitudinal alignment and the intermediate closed-cell ring 32c interconnected to each of the distal and proximal Z-rings 34z, 36z by four longitudinally extending linear interconnectors T_{d}, Tₚ, of the type shown in Fig. 5(a), respectively, interconnecting every third pair of opposed peaks of the adjacent annular rings. The interconnectors Tₚ interconnecting the proximal side of the intermediate closed-cell ring 32c to the proximal Z-ring 36z are situated circumferentially midway between each adjacent pair of interconnectors T_{d} interconnecting the distal side of the intermediate closed-cell ring 32c to the distal Z-ring 34z in order to facilitate a uniform expansion of a stent incorporating module 24.

A stent constructed from a plurality of modules 24, unlike a stent formed from a plurality of modules 10 (Fig. 9), will provide a high degree of radial force along relatively short axial length portions, and relatively high flexibility and kink resistance along its length, due to the repetition of Z-segments comprising pairs of longitudinally adjacent Z-rings (at the connected ends of adjacent modules) separated by closed-cell segments, each only of a single closed-cell ring. Such a stent is useful in treating a relatively short stenosis situated at or near the apex of a substantially curved vessel or in a tortuous vessel. The use of linear interconnector elements Tₚ and T_{d} in the manner shown and described provides the stent with additional flexibility and uniform expansion characteristics. As in the case of module 10, the particular configuration of the closed-cell rings and the specific manner of interconnection between longitudinally adjacent rings can be varied from that shown in Fig. 10.

A stent constructed from a plurality of interconnected Type B module in general will have good flexibility along its length while the length of the stent providing a high radial force can be increased by adding additional closed-cell rings to closed-cell segments of the modules.

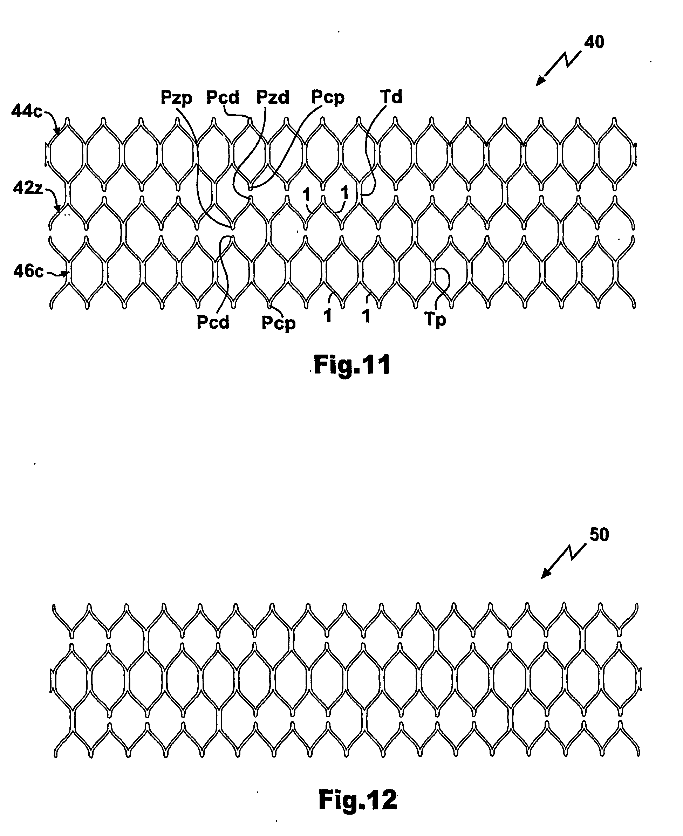

Referring to Fig. 11, a Type A stent module 40 similar to the Type A module 10 shown in Fig. 9 is illustrated. Stent module 40 differs from module 10 in that each of the annular rings, namely, intermediate Z-ring 42z and distal and proximal closed-cell rings 44c and 46c, define sixteen distal and proximal peaks P(c)_{d,}P(c)ₚ; P(z)_{d}, P(z)ₚ; P(c)_{d}, P(c)ₚ. Opposed, longitudinally aligned peaks P(z)ₐ, P(c)ₚ, of adjacent rings 42z and 44c are interconnected by four longitudinally extending linear interconnectors T_{d} interconnecting every fourth pair of opposed peaks while longitudinally aligned peaks P(z)ₚ, P(c)_{d} of adjacent rings 42z and 46c are interconnected by four longitudinally extending linear interconnectors Tₚ interconnecting every fourth pair of opposed peaks. If the length of the struts 1 forming the Z-rings and closed-cell rings of the module 40, and the angle α at the bends of Z-rings and closed-cells, are the same as the length of the struts 1 and angles forming the rings of module 10, the diameter of a stent incorporating module 40 will be greater than the diameter of a stent incorporating module 10. On the other hand, if the length of struts 1 forming the rings of module 40 is reduced with a constant angle at the bends, or if the length of the struts 1 is the same, but the angle of the bends is reduced, compared to module 10, so that the diameter of the stent incorporating module 40 will be the same as the diameter of a stent incorporating module 10, an axial portion of a stent incorporating module 40 will provide an increased coverage or scaffolding, i.e. an increased metal to vessel wall ratio, compared to an axial portion of a stent incorporating module 10.

Likewise, the Type B stent module 50 shown in Fig. 12 is similar to the Type B module 24 shown in Fig. 10, differing only in the number of peaks defined by the annular rings (16 versus 12) and the spacing of the interconnectors (every fourth pair versus every third pair). In the case where the struts 1 of stent module 50 are the same length as the struts 1 of stent module 24 (Fig. 10), and the angle at the bends is also the same as in stent module 24, the diameter of the axial stent portion incorporating module 50 will be greater than the diameter of a stent portion incorporating module 24 whereas if the struts are shorter with the constant angle, or if the struts are the same, but the angles reduced, compared to module 24, the metal to vessel wall ratio is increased.

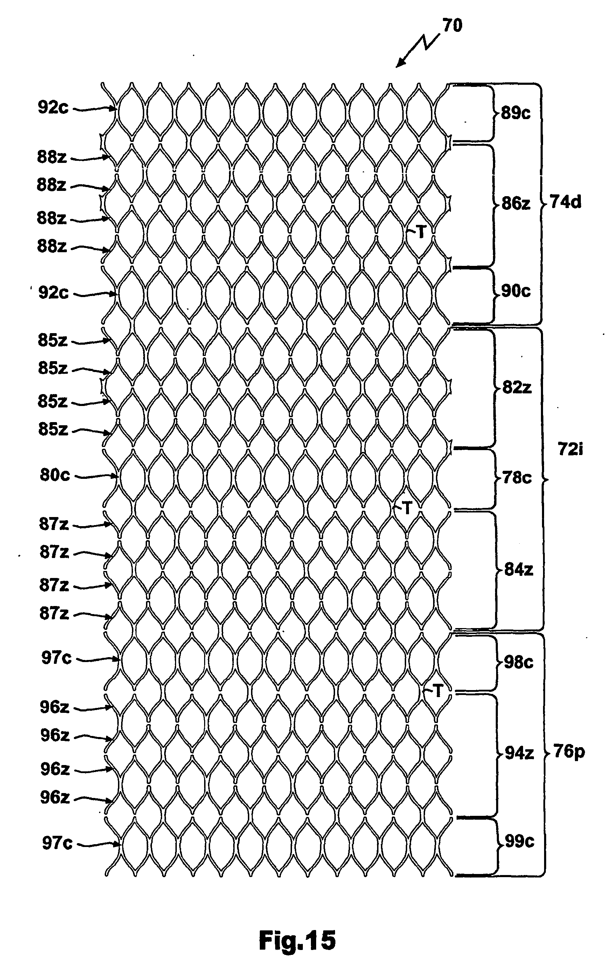

Referring to Fig. 15, a stent 70 in accordance with the present invention is illustrated comprising three stent modules, namely, an intermediate Type B module 72i and distal and proximal Type A end modules 74_{d} and 76ₚ. The stent and its modules are formed of annular rings, each having twelve distal and proximal peaks, which are longitudinally aligned with the distal and proximal peaks of longitudinally adjacent annular rings interconnected by linear interconnectors T situated at every third pair of aligned peaks.

The intermediate Type B module 72i of stent 70 comprises an intermediate closed-cell segment 78c consisting of a single closed-cell ring 80c, distal and proximal Z-segments 82z and 84z, the distal Z-segment 82z consisting of four Z-rings 85z and the proximal Z-segment 84z consisting of four Z-rings 87z,

The distal Type A module 74_{d} comprises an intermediate Z-segment 86z consisting of four Z-rings 88z and a pair of distal and proximal closed-cell end segments 89c and 90c, each consisting of a single closed-cell ring 92c. Similarly, the proximal Type A module 76p comprises an intermediate Z-segment 94z consisting of four Z-rings 96z and a pair of distal and proximal closed-cell end segments 98c and 99c, each consisting of a single closed-cell ring 97c.

The stent 70 has good flexibility and kink resistance and is particularly useful for long irregular lesions situated in a curved vessel. The relatively long Z-segments 82z, 84z, 86z and 94z, each comprising four Z-rings, provide good flexibility along the entire length of the stent, while the closed-cell segments 78c, 89c, 90c, 98c and 99c, each constituted by a single closed-cell ring, provide high radial force and good kink resistance along uniformly spaced intervals of the length of the stent 70. The provision of a single closed-cell ring 80c at the center and more peripheral closed-cell rings 92c and 97c of the stent provide good kink resistance when the stent is bent at these segments through small radius curved vessels.

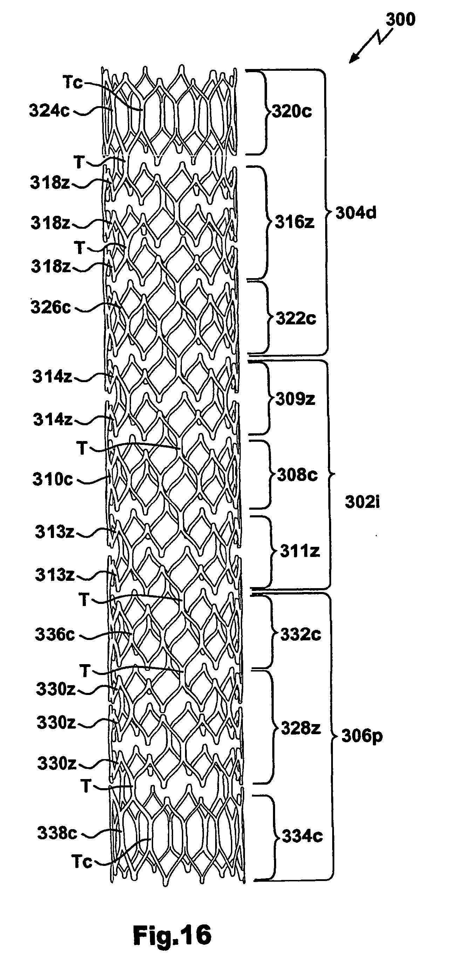

Referring now to Figs. 16-18, a stent 300 in accordance with the invention is illustrated which is capable of treating a wide variety of lesions, and which provides good wall apposition in tortuous vessels and superior resistance to kinking when situated in vessels having acute bends.

The stent 300 comprises three stent modules, namely, an intermediate Type B module 302i, and distal and proximal Type A end modules 304d and 306p. The intermediate module 302i includes an intermediate closed-cell segment 308c constituted by a single closed-cell ring 310c, and distal and proximal Z-segments 309z and 311z, the distal Z-segment 309z consisting of two Z-rings 314z and the proximal Z-segment 311z consisting of two Z-rings 313z.

The distal Type A module 304d comprises an intermediate Z-segment 316z consisting of three Z-rings 318z and distal and proximal closed-cell end segments 320c and 322c, each of which consists of a single closed-cell ring 324c and 326c respectively. Similarly, the proximal Type A module 306p comprises an intermediate Z-segment 328z consisting of three Z-rings 330z and distal and proximal closed-cell segments 332c and 334c, each of which consists of a single closed-cell ring 336c and 338c.

The annular rings forming stent 300 each define twelve distal and proximal peaks, and the rings are arranged with their opposed peaks in longitudinal alignment with each other. The rings are interconnected by linear interconnectors T extending between every third pair of opposed peaks.

The closed-cell rings are of the hexagonal cell type shown in Fig. 2(a) but it is understood that other closed-cell ring configurations may be used to obtain the particular characteristics associated with those configurations as discussed above. In this connection, it is noted that the most distal and proximal rings of stent 300, namely, closed-cell rings 324c and 338c are elongated compared to the other closed-cell rings of stent 300. In other words, the interconnectors T_{c} interconnecting the Z-ring components forming the closed-cell rings 324c and 338c are longer than the interconnectors T interconnecting the rings of the remainder of the stent. By this construction, the distal and proximal closed-cell rings provide a greater stability at the ends of the stent for better anchoring and improved stabilization of the stent in the vessel. It is understood that the end closed cells do not necessarily have to be different in size, compared to other closed-cell segments within the same stent. For example, referring to Fig. 16, the end closed-cells 324c and 338c can be the same in size as closed-cells 326c, 310c and 336c.

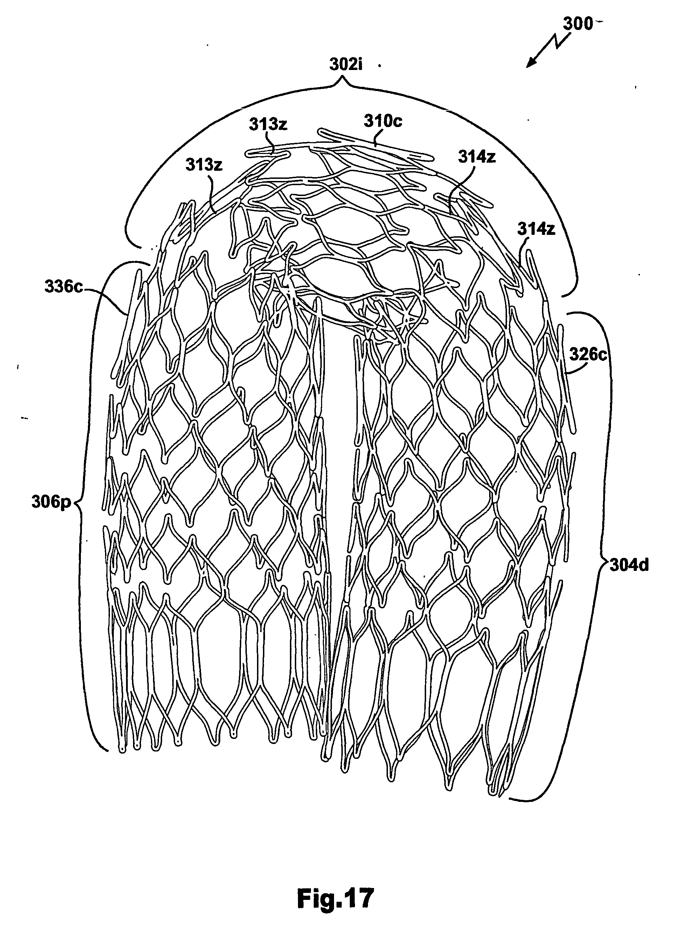

With reference to Fig. 17, it is common for lesions to be located in curved vessel segments which can be extremely tortuous thereby requiring the stent to provide additional features specific to such applications. In this connection, a common and significant problem in the case where a lesion is located at or near a small radius curved vessel segment arises from a decrease of wall coverage and radial force of struts of Z-rings of conventional stents in the region of the curvature. At the same time, an additional problem arises in the region of the inner vessel curvature where the struts of Z-rings of conventional stents overlap each other. Referring to Fig. 17, by providing the intermediate module 302i as a Type B module with only a single closed-cell ring 310c constituting the intermediate module segment, and a pair of Z-segments, each consisting of two Z-rings 313z, 314z, adjacent to the closed-cell ring the stent can be bent up to 180° with the region of the apex of the stent providing good vessel wall coverage and higher radial force. With this construction, the overlap of adjacent struts in the inner region of the apex of curvature of the stent is also reduced. Moreover, this construction provides good wall support in areas of the vessel adjacent to the apex of curvature in cases where the stenosis is not at the region of curvature but close to it by virtue of closed-cell rings 326c and 336c. Thus, despite the accentuated acute angle, the overall integrity of the inner stent lumen is preserved, with relatively little effect on wall coverage, radial force and strut overlap.

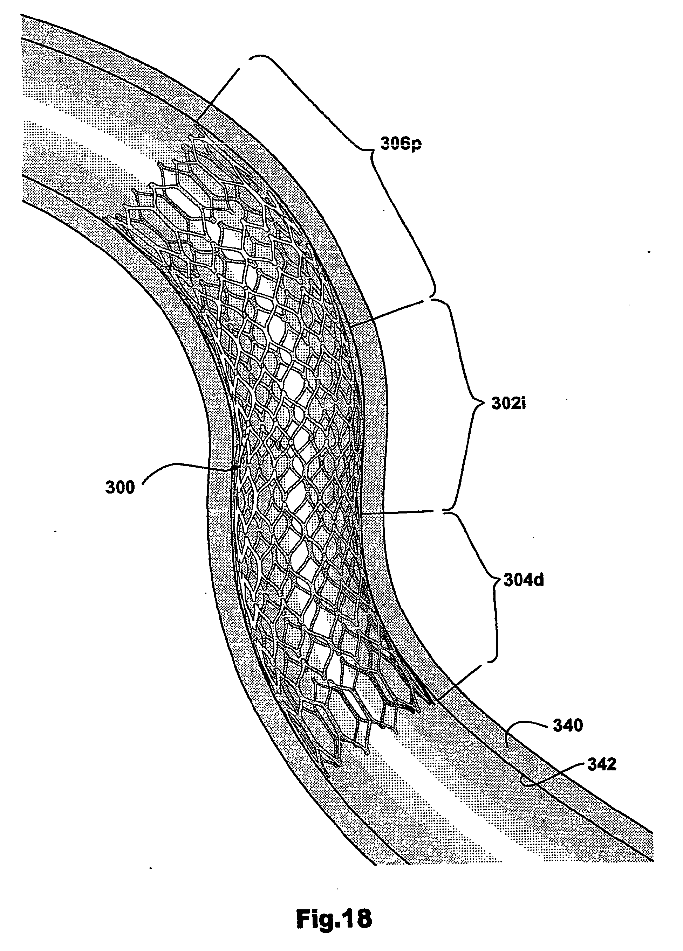

Further, referring to Fig. 18, the stent 300 is shown positioned in an S-shaped moderately tortuous vessel 340. The close apposition of the stent 300 to the vessel wall 342 along the tortuous vessel segment, which is provided by the particular "A-B-A" modular construction, and the high degree of flexibility inherent therein, is illustrated.

It is also possible to form the stents of the invention in a tapered configuration, such as for use in a narrowing vessel, by expanding the small diameter tubes on tapered mandrels. For example, referring to Fig. 27, a tapered stent 300T is illustrated having the same sequence and configuration of closed-cell rings and Z-rings as stent 300 of fig. 16.

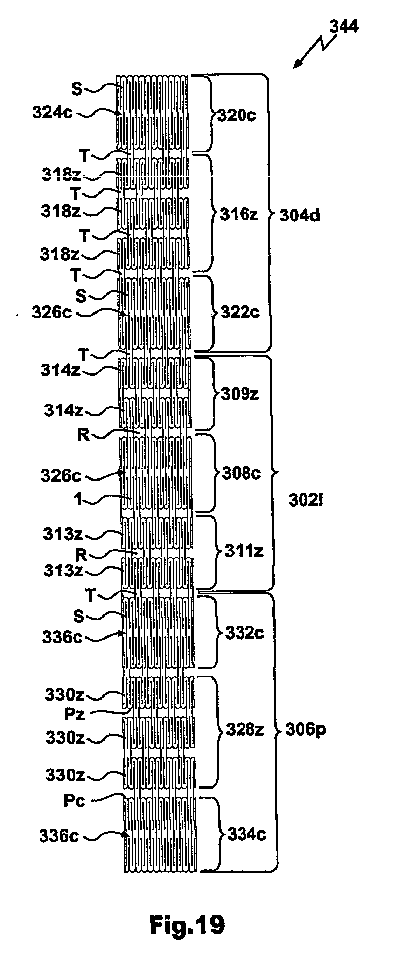

Referring to Fig. 19, a laser-slotted tube 344 is shown which constitutes a stent similar in configuration to stent 300, but with the end closed-cells identical in size to the central closed-cells, and shown in its unexpanded configuration, cut longitudinally and flattened. The tube 344 is constructed of a very thin cylinder of nickel-titanium alloy approximately 0.15-0.30 mm in thickness. The tube is about 1.6-1.8 mm in diameter. A computer controlled laser beam cuts a series of slots S through the tube 344 as well as cutout regions R cooperating with the slots to define the struts 1 and interconnectors T. In manufacture, the tube 344 is progressively expanded by fitting it over mandrels of progressively increasing diameters so that the struts circumferentially open at their points of intersection to define the peaks and valleys. The stent is heat treated after each expansion to impart to it the desired temperature sensitive memory characteristics, with the final expansion and heat treatment step determining the final diameter of the stent in its expanded configuration. In the case of the laser-slotted tube 344, the tube is expanded from 1.8 mm to 12 mm in several steps. Any one of the intermediate steps can be a final step if a smaller final stent diameter is indicated for a particular application.

The tube 344 has a length of about 6 cm, although it is understood that the tube can be cut to any desired length. With a length of 6 cm, the stent can be used in treating a stenosis up to about 4 cm in length, in which case the stenosis should be centered along the length of the stent so that the ends of the stent, including closed-cell rings 324c and 338c, appose healthy regions of the vessel wall to anchor the stent in place.

The five closed-cell segments 320c, 322c, 308c, 332c and 334c spaced at intervals over the length of the stent provide a relatively uniform high radial force distribution over the length of the stent so that the stent can be used to treat from very short to very long stenoses with assurance that a high radial force will be applied by the stent. At the same time, the four Z-segments 316z, 309z, 311z and 328z situated between the closed-cell segments provide good flexibility along the length of the stent to enable the stent to be used in tortuous vessels as shown in Fig. 18. The particular numbers of closed-cell rings and Z-rings in each closed-cell and Z-segment of stent are chosen in this embodiment to provide uniform, high radial force distribution and flexibility for a large variety of types and lengths of stenoses and a high degree of longitudinal flexibility, both in its unexpanded configuration shown in Fig. 19 as well as in its expanded configuration shown in Fig. 18. Moreover, the provision of the three central single-ring closed-cell segments 326c, 310c and 336c, each of which is bounded on its distal and proximal ends by Z-segments incorporating two or more Z-rings, provides the stent with good kink resistance characteristics along its length. As seen in Fig. 17, which shows the stent 300 bent about 180° at its central region, the single closed-cell ring 310c will provide good radial support at the apex of the bend, while the struts of the closed-rings 313z, 313z, 314z, 314z only slightly overlap thereby allowing the stent lumen to remain open for flow. Similar resistance to kinking will be obtained if stent 300 is bent at its end regions proximate to single-ring closed-cell segments 326c and 336c.

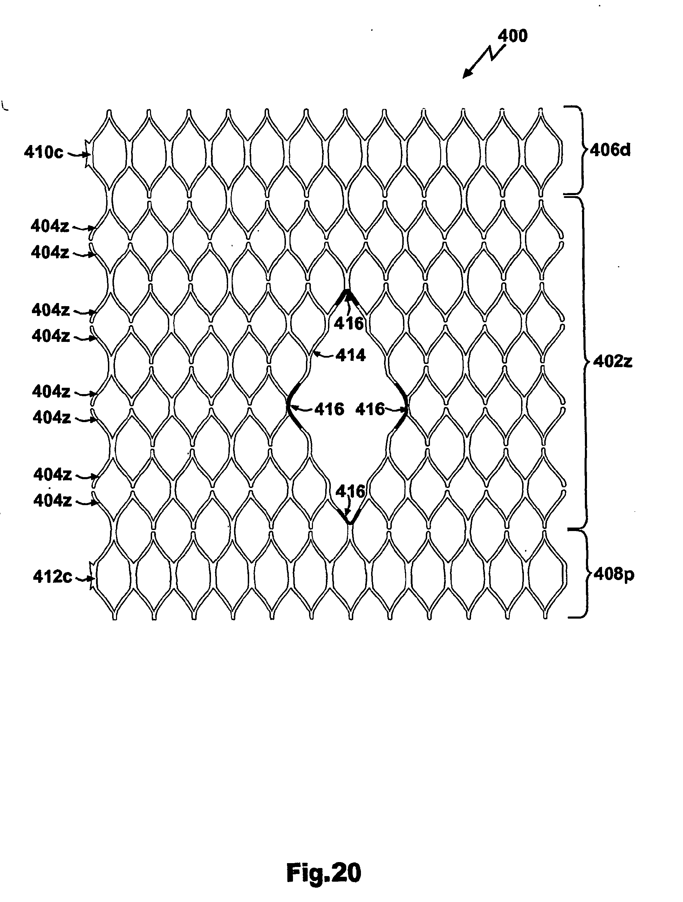

Referring to Fig. 20, a stent module 400 is illustrated, cut longitudinally and flattened, particularly suitable for use in a stent for a vessel in which a lesion is situated at or near a side branch to that vessel. The module 400 comprises a Type A module including an intermediate Z-segment 402z consisting of eight Z-rings 404z and distal and proximal closed-cell end segments 406d and 408p, each consisting of a single closed-cell ring 410c and 412c respectively. A relatively large irregular diamond-shaped cell or window 414 is formed centrally in the intermediate Z-segment 402, having a length extending over six Z-rings and a width extending at its widest point over four Z-ring peaks, through which the end of another stent situated in the side branch to the vessel can be received. The four corners of the window 414 are coated with radio-opaque material 416 in order to assist the clinician in accurately positioning the stent so that the window is situated at the side branch.

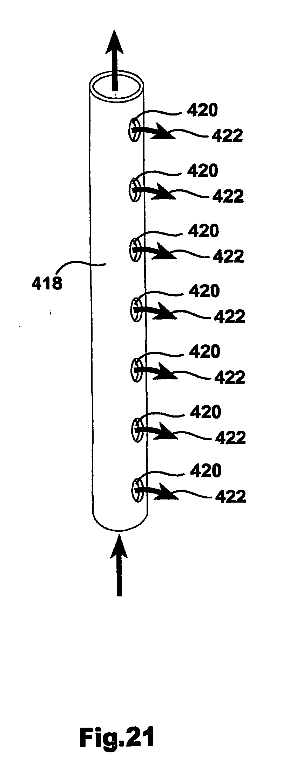

The stents can be formed of thin, porous fenestrated micro-tube elements of the type schematically shown in Fig. 21. The porous nature of micro-tube element 418 is schematically indicated at 420. The stent tube elements are filled with medication, usually in gel form, prior to delivery so that once the stent is delivered and expanded, the medication will gradually release, shown schematically at 422, into the blood stream or into the vessel wall for treatment of a particular condition, or to prevent restenosis at the site of stent deployment.

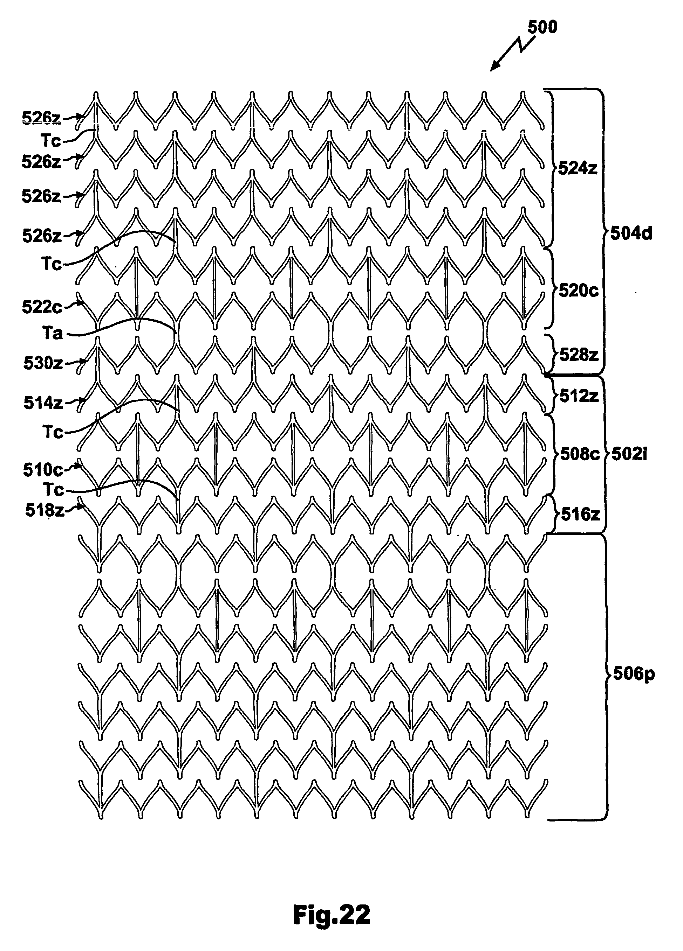

Referring to Fig. 22, another preferred embodiment of a stent, designated 500, in accordance with the present invention is shown in its expanded configuration, cut longitudinally and flattened. Stent 500 exhibits minimal foreshortening upon expansion and provides high radial strength at its closed-cell segments and a high degree of flexibility at its Z-segments.

Stent 500 comprises an intermediate Type B module 502i interconnected at its distal and proximal ends to Type B end modules 504d and 506p. Intermediate module 502i comprises an intermediate closed-cell segment 508c consisting of a single closed-cell ring 510c, a distal Z-segment 512z consisting of a single Z-ring 514z and a proximal Z-segment 516z consisting of a single Z-ring 518z. Closed-cell ring 510c is of the type shown in Fig. 2(d) thereby providing this portion of the stent with reduced foreshortening characteristics with only slightly less radial force and flexibility than closed-cell rings of the type shown in Fig. 2(a). The distal and proximal Z-rings 514z, 518z are longitudinally offset with respect to intermediate closed-cell ring 510c and are interconnected to the closed-cell ring 510c by interconnectors T_{c} in accordance with the arrangement shown in Fig. 5(c).

The distal end module 504d comprises an intermediate closed-cell segment 520c consisting of a single closed-cell ring 522c, again of the Fig. 2(d) type, a distal Z-segment 524z consisting of four Z-rings 526z interconnected to each other and to closed-cell ring 522c by interconnectors T_{c}, again in accordance with the Fig. 5(c) arrangement, and a proximal Z-segment 528z consisting of a single Z-ring 530z. The closed-cell ring 522c is interconnected to the Z-ring 530z by interconnectors Tₐ of the type shown in Fig. 5(a). The proximal end module 506p is essentially a mirror-image of the distal end module 504d.

By using closed-cell rings of the Fig. 2(d) type in all three modules and interconnectors T_{c} of the Fig. 5(c) type, the stent 500 will exhibit a minimum degree of foreshortening upon expansion and relative high radial strength along the length of the stent. The long Z-segments of the distal and proximal end modules 504d, 506p, comprising four Z-rings, provide the stent with good flexibility at its ends. Reduced foreshortening of the stent upon expansion allows a more accurate positioning of the stent at the desired location of the vessel.

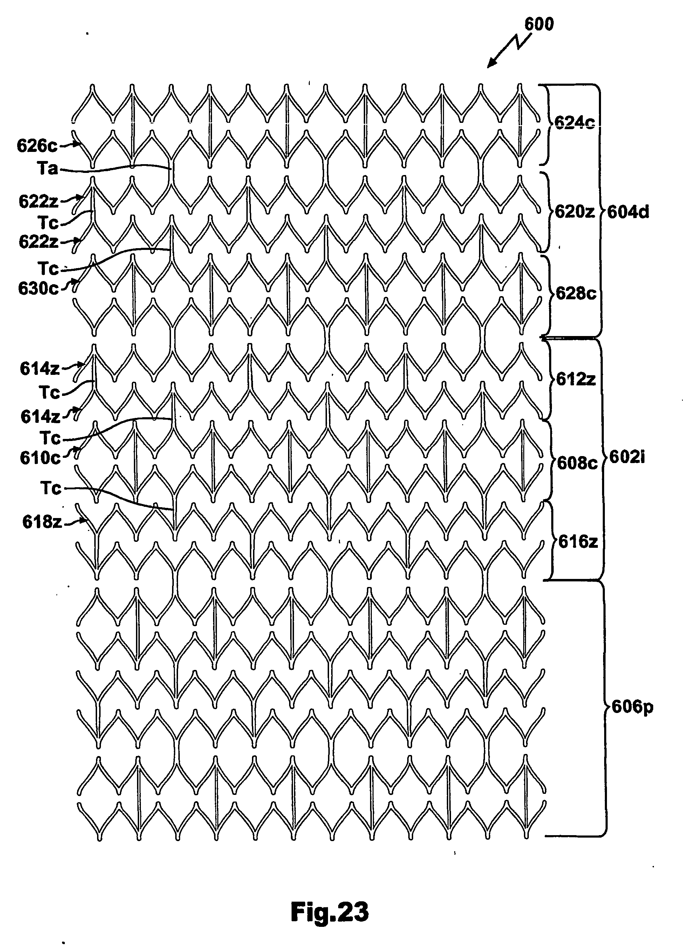

Referring to Fig. 23, another preferred embodiment of a stent, designated 600, in accordance with the present invention is shown in its expanded configuration, cut longitudinally and flattened. Like stent 500, stent 600 will exhibit a minimum degree of foreshortening upon expansion.

Stent 600 comprises an intermediate Type B module 602i interconnected at its distal and proximal ends to Type A end modules 604d and 606p. Intermediate module 602i comprises an intermediate closed-cell segment 608c consisting of a single closed-cell ring 610c, a distal Z-segment 612z consisting of two Z-rings 614z and a proximal Z-segment 616z consisting of two Z-rings 618z. As in the case of stent 500, closed-cell ring 610c is of the Fig. 2(d) type providing reduced foreshortening characteristics. The proximal and distal Z-rings 614z, 618z are interconnected to the intermediate closed-cell ring 610c by interconnectors T_{c} of the Fig. 5(c) type.

The distal end module 604d comprises an intermediate Z-segment 620z consisting of two Z-rings 622z interconnected by Fig. 5(c) type interconnectors T_{c}, separated by four pairs of opposed peaks and valleys, a distal closed-cell segment 624c consisting of a single closed-cell ring 626c of the Fig. 2(d) type and connected to the adjacent Z-ring 622z by Fig. 5(a) type interconnectors Tₐ, and a proximal closed-cell segment 628c consisting of a single closed-cell ring 630c of the Fig. 2(d) type and connected to the adjacent Z-ring 622z by a Fig. 5(c) type interconnector T_{c}. The proximal end module 606ₚ is essentially a mirror image of the distal end module 604_{d}. The stent 600 also exhibits minimum foreshortening upon expansion due to the combination of the Fig. 5(a) and Fig. 5(c) type interconnectors and Fig. 2(d) type closed-cell rings.

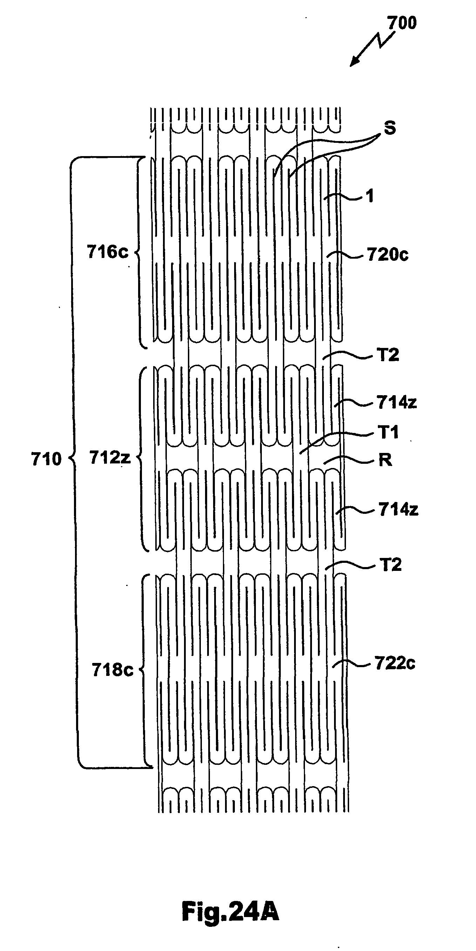

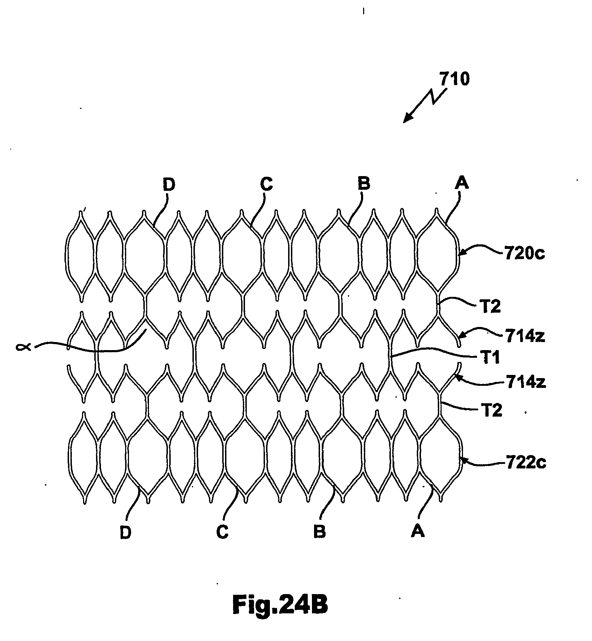

Referring now to Figs. 24(a) and 24(b), as non-uniform, multi-segment stents are manufactured from thinner laser-slotted tubes with narrower struts, problems may arise with achieving geometric regularity in the stent construction in its expanded configuration.

Specifically, an end portion of a small diameter laser-slotted tube 700 formed of a nickel-titanium alloy for producing a self-expandable stent is shown in Fig. 24(a), cut longitudinally and flattened. The end portion of the laser-slotted tube embodies a stent module 710 including an intermediate Z-segment 712z consisting of two Z-rings 714z interconnected by interconnectors T₁ and distal and proximal closed-cell segments 716c and 718c consisting of single closed-cell rings 720c and 722c respectively which are connected to Z-rings 714z by interconnectors T₂.

In the manufacture of a self-expanding stent, slots S and cutout regions R are initially cut in the tube 700 by a laser beam to define the struts and interconnectors. The tube is then progressively mechanically expanded and heat treated in several steps, such as by applying the slotted tube over mandrels of progressively increasing diameter, until the tube is expanded to its final diameter. In this connection, the tube 700 in this embodiment having an initial diameter of 1.8 mm can be mechanically expanded in progressive steps to different final diameters, e.g., 6 mm, 8 mm or 12 mm, depending upon the actual application to which the stent will be put.

Referring to Fig. 24(b), when the stent is formed with annular rings of different geometry, e.g. Z-rings and closed-cell rings 720c, 714z, 722c, and, especially when the tube 700 is formed of thin material, e.g. 0.16-0.22 mm, and the struts 1 formed by the slots S are narrow, e.g., less than about 0.2 mm, the stent will tend to expand during manufacture in an irregular manner, i.e. with the cells of each closed-cell ring and the Z-shaped portions of each Z-ring having an irregular or distorted geometry. This is due to some struts being situated closer to interconnectors than others and being constrained against movement to a greater extent than those struts situated further from those regions. For example, referring to Fig. 24(b) which illustrates the stent module 710 expanded to a diameter of 6 mm, the closed-cells A, B, C and D in closed-cell rings 720c and 722c, the peaks of which are connected to the Z-rings 714z by interconnectors T₂, are circumferentially wider than the other closed-cells in those rings. Similarly, the configuration of the Z-shaped portions of Z-rings 714z are irregular and distorted around the circumference of each of the Z-rings. If those geometric irregularities remain in the final stent, which is likely in the case of stents having relatively small final diameters, the stent will not provide uniform radial force and will not provide good wall apposition, as well as uniform metal-to-wall ratio circumferentially, with larger cells allowing protrusion of atherosclerotic material into the vessel lumen.

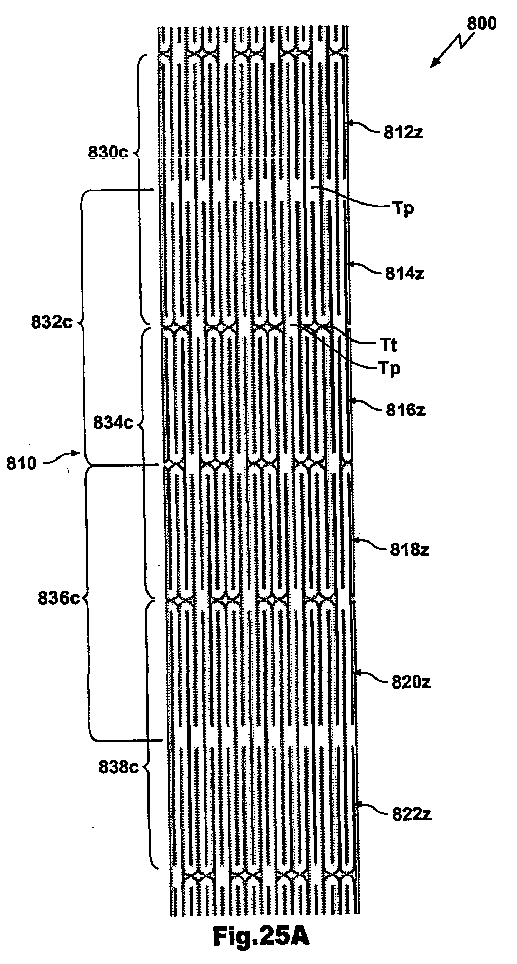

Referring to Fig. 25(a) in which the end portion 810 of a laser-slotted small diameter tube 800 is shown, this problem is overcome by laser-cutting the small diameter tube 800 to define a plurality of longitudinally adjacent Z-rings 812z, 814z, 816z, 818z, 820z, 822z, each Z-ring having a plurality of peaks and valleys, and providing temporary and permanent interconnector portions, Tₜ and Tₚ, integrally joining adjacent pairs of Z-rings so that every pair of adjacent Z-rings constitutes, at least temporarily, a closed-cell ring. Thus, adjacent Z-rings 812z and 814z constitute a closed-cell ring 830c, adjacent Z-rings 814z and 816z constitute a closed-cell ring 832c, adjacent rings 816z and 818z constitute a closed-cell ring 834c, adjacent rings 818z and 820c constitute a closed-cell ring 836c and adjacent rings 820z and 822z constitute a closed-cell ring 838c.

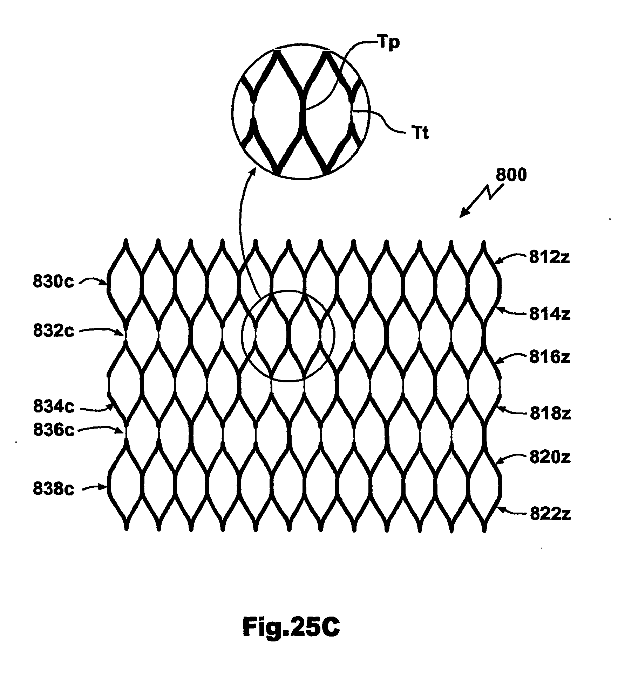

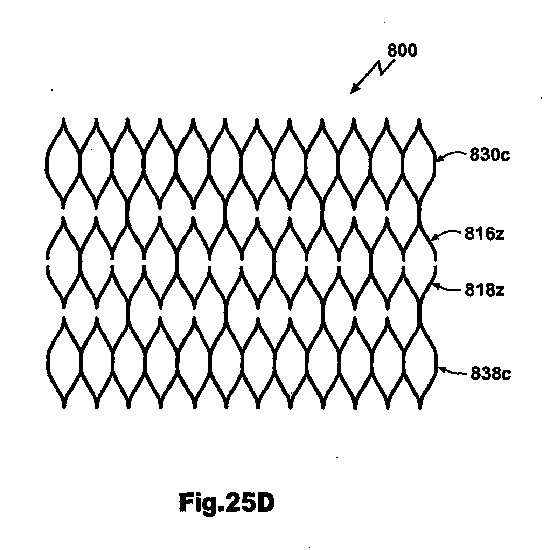

The small diameter tube is then expanded and heat treated to its final diameter as seen in Fig. 25(c) whereupon the temporary interconnectors T_{T} are removed from the expanded tube (Fig. 25(d)), either mechanically or by laser-cutting, to provide the desired sequence and arrangement of closed-cell and Z-rings, namely 830c, 816z, 818z, 838c. The expanded tube is then electro-polished to smoothen all surfaces. Each of the closed-cell rings and Z-rings has a regular, non-distorted configuration since the temporary interconnectors T_{T} functioned to constrain the opposed peaks of adjacent annular rings to remain in uniform regular relationship during the expansion step.

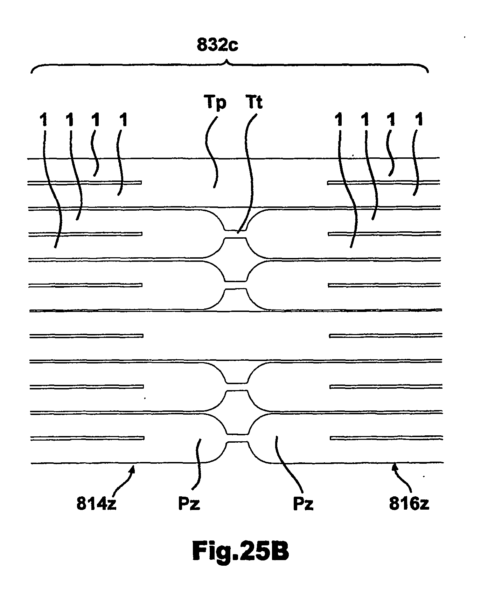

As best seen in Fig. 25(b), the temporary interconnectors T_{T} of tube 800 constitute thin webs of tube material integrally joining pre-shaped peaks P(z)ₚ, P(z)_{d} of adjacent Z-rings, e.g. Z-rings 814z and 816z. Each permanent interconnector Tₚ essentially constitutes a continuation of a respective pair of struts forming the opposed peaks of adjacent Z-rings which remain interconnected after expansion of the tube and removal of the temporary interconnectors Tₚ. Thus, as seen in Fig. 25(c), after the tube 800 has been expanded, the temporary struts T_{T} are significantly thinner than the permanent struts Tₚ and are easily identified for mechanical removal.

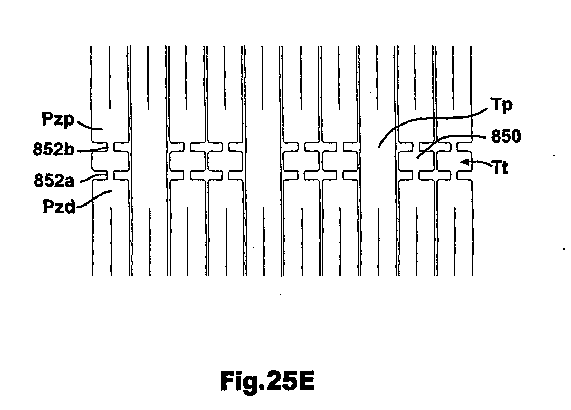

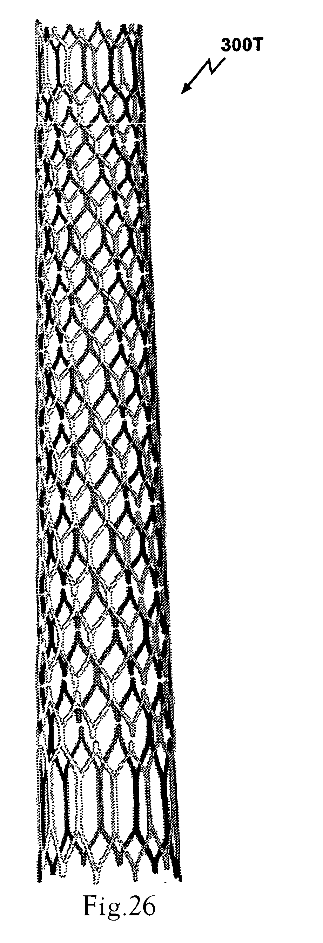

The temporary interconnectors T_{T} can have other forms than that shown in Figs. 25(a) and 25(b). For example, as shown in Fig. 25(e), in order to facilitate manual removal, the temporary interconnectors T_{T} can be formed in the tube 800 to include enlarged flag portions 850 joined to respective opposed aligned peaks P(z)_{d} and P(z)ₚ by very short, thin connecting portions 852a and 852b. These enlarged flag portions are easily grasped by a tool to facilitate removal after the tube has been expanded to its final diameter.

Referring now to Figs. 27-29, it is often costly and time-consuming to create specific software for guiding the laser-cutting tool to cut a particular desired sequence and arrangement of closed-cell rings and Z-rings in a small diameter tube to provide a stent with optimal characteristics for a particular clinical application. Stent blanks are initially prepared, which may be done before particular clinical application of the stent is known, and therefore before the desired sequence and arrangement of the Z-rings and closed-cell rings has been determined, from which a stent having any desired sequence and arrangement can be simply and quickly made. Thus, a manufacturer may maintain an inventory of stent blanks so that when a patient requires a stent for a particular application, e.g.; for use in a tortuous vessel where the stenosis is situated in a small radius curved portion, a clinician can request a stent having the sequence and configuration of Z-rings and closed-cell rings particularly suited for that application, which can be simply and quickly made from one of the stent blanks, loaded into the delivery system, sterilized and shipped to the hospital.

A stent blank is formed by laser-cutting a small diameter tube to define a plurality of longitudinally adjacent Z-rings having interconnector portions integrally joining adjacent Z-rings in a manner such that every pair of adjacent Z-rings constitutes a closed-cell ring. The small diameter tube is then expanded and heat-treated to form the stent blank. Once the particular desired sequence and arrangement of closed-cell rings and Z-rings is determined, certain interconnector portions are removed from the blank, either mechanically or by laser, to provide the desired stent configuration.

Referring to Fig.27(a), a stent blank 80 manufactured by expanding and heat treating a small diameter tube of shape-memory material is shown. The blank 80 is formed of a plurality of Z-rings 82 situated in aligned relationship with each other with each pair of proximate aligned peaks P(z) being interconnected by interconnector portions T, so that every pair of adjacent Z-rings 82 forms a closed-cell ring 84 in the manner described in connection with Fig. 2(a). Closed-cell rings 84 are interconnected by shared walls in the manner described in connection with Fig. 8.

Stents having a wide variety of sequences and arrangements of closed-cell rings and Z-rings suitable for different clinical applications can be made simply and quickly from blanks 80. For example, where a stenosis requires a uniform radial force distribution along an extended length, the practitioner may determine that a stent having the following sequence of closed-cell rings and Z-rings will be optimal: CCZZC ZZCZZ CZZCC, where C designates a close-cell ring and Z designates a Z-ring.

Referring to Fig. 27(b), a blank 80 is quickly and simply processed to form a stent 85 having this configuration by removing selected interconnector portions T from between selected pairs of adjacent Z-rings. For example, beginning at the distal end of stent 85, closed-cell rings 84a and 84b are "formed" having the configuration and properties of closed-cell ring 200a in Fig. 2(a) by leaving, i.e. not removing, the interconnectors Ta, Tb that join every pair of proximate aligned peaks of adjacent pairs of Z-rings 82₁, 82₂ and 82₂, 82_{3.} The Z-ring 82₃ is formed having the configuration and properties of the Z-rings 100 shown in Fig. 5(a) by removing pairs of interconnectors Tc joining circumferentially adjacent pairs of proximate aligned peaks, i.e. leaving the interconnectors Tc that join every third pair of proximate aligned peaks of adjacent Z-ring pair 82_{3,} 82_{4.} The formation of the remainder of stent 85 is apparent from the foregoing.

Not only can any desired sequence of closed-cell rings and Z-rings be obtained using the blank 80, but the arrangement of each ring itself can be varied. For example, the closed-cell ring 84a can be formed by removing the interconnectors Ta joining every other pair of proximate aligned peaks so that the closed-cell ring will have the configuration and properties of closed-cell 200b shown and described in Fig. 2(b).

Fig. 27(c) illustrates a stent 85 made from the stent blank 90 having the following sequence of closed-cell rings and Z-rings: ZZZCZZ ZCCCZ ZZCZZZ, which may be desired where a high radial force is needed along a relatively short length of a tortutous vessel.

Fig. 28(a) illustrates another stent blank 87. The stent blank 87 comprises a plurality of Z-rings 88 situated in aligned relationship to each other with interconnectors T joining every pair of proximate aligned valleys to form closed-cell rings 89, each having the configuration and properties of the closed-cell ring 200c of Fig. 28(c). Stents formed from blank 87 generally will exhibit a lesser degree of foreshortening upon expansion than stents formed from blank 80 of Fig. 27(a).

Fig. 28(b) illustrates a stent 90 made by removing appropriate interconnectors from the stent blank 87 and having the following sequence of closed-cell rings and Z-rings: CCZZZC ZCZ CZZZCC, which may be desired for certain applications apparent from the foregoing. In this case, closed-cell rings are formed by leaving interconnectors T joining every pair of proximate aligned valleys of adjacent Z-rings while Z-rings are formed by removing interconnectors T joining circumferentially adjacent pairs of proximate aligned valleys, i.e., leaving the interconnectors T that join every third pair of proximate aligned valleys.

Fig. 28(c) illustrates a stent 91 made by removing appropriate interconnectors from the stent blank 87 and having the following sequence of closed-cell rings and Z-rings: CCZZZC ZCZ CZZZCC which may be desired for certain applications apparent from the foregoing. It is noteworthy that some of the closed-cell rings C, e.g. ring C₁, of stent 91 are formed by leaving interconnectors T joining every pair of proximate aligned valleys, while other closed-cell rings C, e.g. ring C₂, of stent 91, are formed by removing interconnectors T from every other pair of proximate aligned valleys.

Fig. 28(d) illustrates a stent 91 A made from stent blank 87 and having the following sequence of closed-cell rings and Z-rings: ZZZCZ ZZCCCZZ ZCZZZ.

Fig. 29(a) illustrates a third stent blank 93. The stent blank 93 comprises a plurality of Z-rings 94 situated in offset relationship to each other (except for Z-rings 94ₐ and 94_{b} which are in alignment with each other), with interconnectors T joining every proximate aligned peak and valley pair to form closed-cell rings 95, each having the configuration and properties of the closed-cell ring 200e or 200h of Figs. 2(e) and 2(h). Closed-cell rings 94a and 94b and interconnectors Ta from a closed-cell ring 95a having the configuration and properties of the closed-cell ring 200c of Fig. 28(c). Stents formed from blank 93 generally will exhibit a lesser degree of foreshortening, but similar flexibility and kink resistance compared to stents formed from blank 80.

Fig. 29(b) illustrates a stent 96 made by removing appropriate interconnectors from the stent blank 93 having the following sequence of closed-cell rings and Z-rings: CZZZC ZZCZZ CZZZC. Similarly, Fig. 29(c) illustrates another stent 97 made from stent blank 93 having the following sequence of closed-cell rings and Z-rings: ZZZCCZZ ZCZ ZZCCZZZ.

It is also possible to form a stent blank, prior to determining the sequence and arrangement of the closed-cell rings and Z-rings, from an enlarged diameter tube by laser-cutting the enlarged diameter tube to define a plurality of longitudinally adjacent Z-rings which are interconnected by interconnector portions such that every pair of adjacent Z-rings constitutes a closed-cell ring. Once the particular sequence and configuration of the closed-cell rings and Z-rings has been determined, appropriate interconnector portions are removed as in the previously disclosed methods.

## Claims

1. A modular stent having an unexpanded configuration and an expanded tubular configuration, said stent compromising at least two stent modules (72i, 74d; 502i, 504d) **characterized in that**
at least one stent module (72i; 502i) includes,
an intermediate segment (78c; 508c) consisting of a ctosed-cell segment (78c; 508c);
a pair of end segments (82z, 84z; 512z, 516z) connected to respective longitudinal ends of said intermediate segment (78c; 508c),
each end segment (82z, 84z; 512z, 516z) consistming of a Z-segment (82z, 84z; 512z, 516z),
each Z-segment (82z, 84z; 512z, 516z) consisting solely of at least one annular Z-ring (85z, 87z; 514z, 518z) formed by struts defining an elongate member including a plurality of wave-shaped portions having a plurality of proximal and distal peaks and valleys;
each closed-cell segment (78c, 508c) consisting solely of at least one annular closed-cell ring (80c; 510c) formed by struts defining a plurality of closed-cell elements having a plurality of proximal and distal peaks and valleys; and
at least a second stent module (74d; 504d) includes,
an intermediate segment (86z; 520c) consisting of one of either a closed-cell segment (520c) or a Z-segment (86z);
a pair of end segments (89c, 90c; 524z, 528z) connected to respective longitudinal ends of said intermediate segment (86z; 520c),
each end segment (89c, 90c; 524z, 528z) consisting of the other of said closed-cell segment (89c, 90c) or Z-segment (524z, 528z);
each closed-cell segment (89c, 90c; 520c) consisting solely of at least one annular closed-cell ring (92c; 522c) formed by struts defining a plurality of closed-cell elements having a plurality of proximal and distal peaks and valleys;
each Z-segment (86z; 524z, 528z) consisting solely of at least one annular Z-ring 8z; 526z, 530z) formed by struts defining an elongate member including a plurality of wave-shaped portions having a plurality of proximal and distal peaks and valleys.

2. The modular stent according to claim 1 wherein each closed-cell ring (80c) is formed by a pair of opposed Z-rings tightly interconnected at pairs of facing peaks or pairs of facing valleys or pairs of facing peaks and valleys.

3. The stent according to claim 1, wherein a plurality of circumferentially spaced apart connecting members (T) interconnect each pair of axially adjacent Z-ring elements (100d, 100p), and wherein not all proximal and distal valleys of said stent module are interconnected.

4. The modular stent according to any of the above claims wherein each of said annular closed-cell rings (80c) are formed by linear struts.

5. The modular stent according to any of the claims 1 - 3 wherein each of said annular closed-cell rings (80c) are formed by non-linear struts.

6. The modular stent according to any of the claims 1 - 4 wherein each of said annular closed-cell rings (80c) are formed by serpentine-shaped struts.

7. The modular stent according to any of the above claims wherein each of said annular Z-rings are formed by substantially linear struts.

8. The modular stent according to any of the claims 1 - 7 wherein each of said annular Z-rings are formed by serpentine-shaped struts.

9. The modular stent according to any of the claims 1 - 6 wherein each of said annular Z-rings are formed by substantially non-linear struts.

10. The modular stent according to any of the claims 1 - 7 wherein each of said annular Z-rings comprise integral smooth sinusoidal waves.

11. The modular stent according to any of the above claims wherein each closed-cell segment (78c) comprises at least two annular closed cell rings (80c) and interconnectors interconnecting pairs of longitudinally adjacent annular closed-cell rings (80c).

12. The modular stent according to any of the above claims wherein each Z-segment (82z, 84z) comprises at least two annular Z-rings and interconnectors interconnecting pairs of longitudinally adjacent annular Z-rings.

13. The stent according to claim 12, wherein said interconnector interconnects each pair of axially adjacent Z-rings at circumferentially spaced apart locations, and wherein not all locations are uniformly spaced apart.

14. The modular stent according to any of claims 11 to 13 wherein said interconnectors comprise linear interconnectors.

15. The modular stent according to any of claims 11 to 13 wherein said interconnectors comprise serpentine-shaped interconnectors.

16. The modular stent according to any of claims 11 to 13 wherein said interconnectors comprise shared struts of said two interconnected annular closed-cell rings (80c).

17. The modular stent according to any of the above claims wherein each closed-cell segment includes at least one closed-cell ring having from 4 to 16 closed-cell elements defining from 4 to 16 proximal and distal peaks and valleys.

18. The modular stent according to any of the above claims wherein each Z-segment includes at least one Z-ring defining from 4 to 16 distal and proximal peaks and valleys.

19. The modular stent according to any of the above claims wherein said stent is formed of fenestrated wire or porous metallic alloy (418).

20. The modular stent according to any of the above claims wherein said stent has a capability of eluting medications, applied directly to its surface or by means of a polymer or other coating.

21. The modular stent according to any of the above claims wherein said stent is formed of a shape-memory alloy.

22. The modular stent according to claim 21 wherein said shape-memory alloy comprises superelastic nitinol.

23. The modular stent according to claim 21 wherein said stent is formed of tubular material.

24. The modular stent according to claim 21 wherein said stent is formed of a wire material.

25. The modular stent according to any of the above claims wherein said stent is a balloon expandable stent.

26. The modular stent according to any of the above claims having an outermost annular ring at each longitudinal end of the stent and wherein at least one radiopaque marker is applied to at least one of said outermost annular rings of the stent.

27. The modular stent according to claim 1 wherein each closed-cell segment includes from 1 to 4 closed-cell rings, each closed-cell ring having from 4 to 16 closed-cell elements and from 4 to 16 distal and proximal peaks; and
each Z-segment includes from 1 to 8 Z-rings, each Z-ring having from 4 to 16 distal and proximal peaks.

## Patentansprüche

1. Modularer Stent mit einer nicht-expandierten Konfiguration und einer expandierten röhrenförmigen Konfiguration, wobei der Stent zumindest zwei Stentmodule (72i, 74d; 502i, 504d) umfasst, **dadurch gekennzeichnet, dass**
zumindest ein Stentmodul (72i; 502i) Folgendes umfasst
ein Zwischensegment (78c; 508c), bestehend aus einem geschlossenzelligen Segment (78c; 508c);
ein Paar von Endsegmenten (82z, 84z; 512z, 516z), verbunden mit jeweiligen Längsenden des Zwischensegments (78c; 508c),
wobei jedes Endsegment (82z, 84z; 512z, 516z) aus einem Z-Segment (82z, 84z; 512z, 516z) besteht,
wobei jedes Z-Segment (82z, 84z; 512z, 516z) nur aus zumindest einem ringförmigen Z-Ring (85z, 87z; 514z, 518z) besteht, gebildet durch Streben, die ein verlängertes Element definieren, umfassend eine Mehrzahl von wellenförmigen Abschnitten mit einer Mehrzahl von proximalen und distalen Spitzen und Tälern;
wobei jedes geschlossenzellige Segment (78c, 508c) nur aus zumindest einem ringförmigen geschlossenzelligen Ring (80c; 510c) besteht, gebildet durch Streben, die eine Mehrzahl von geschlossenzelligen Elementen mit einer Mehrzahl von proximalen und distalen Spitzen und Tälern definieren; und
zumindest ein zweites Stentmodul (74d; 504d) Folgendes umfasst
ein Zwischensegment (86z, 520c), bestehend aus einem von entweder einem geschlossenzelligen Segment (20c) oder einem Z-Segment (86z);
ein Paar von Endsegmenten (89c, 90c; 524z, 528z), verbunden mit jeweiligen Längsenden des Zwischensegments (86z, 520c),
wobei jedes Endsegment (89c, 90c; 524z, 528z) aus dem Anderen des geschlossenzelligen Segments (89c, 90c) oder des Z-Segments (524z, 528z) besteht;
wobei jedes geschlossenzellige Segment (89c, 90c; 520c) nur aus zumindest einem ringförmigen geschlossenzelligen Ring (92c; 522c) besteht, gebildet durch Streben, die eine Mehrzahl von geschlossenzelligen Elementen mit einer Mehrzahl von proximalen und distalen Spitzen und Tälern definieren;
wobei jedes Z-Segment (86z, 524z, 528z) nur aus zumindest einem ringförmigen Z-Ring (8z; 526z 530z) besteht, gebildet durch Streben, die ein verlängertes Bauteil definieren, umfassend eine Mehrzahl von wellenförmigen Abschnitten mit einer Mehrzahl von proximalen und distalen Spitzen und Tälern.

2. Modularer Stent nach Anspruch 1, bei welchem jeder geschlossenzellige Ring (80c) durch ein Paar von gegenüberliegenden Z-Ringen gebildet ist, die fest miteinander verbunden sind bei Paaren von sich gegenüberliegenden Spitzen oder Paaren von sich gegenüberliegenden Tälern oder Paaren von sich gegenüberliegenden Spitzen und Tälern.

3. Stent nach Anspruch 1, bei welchem eine Mehrzahl von in Umfangsrichtung mit Abstand versehenen Verbindungselementen (T) jedes Paar von axial benachbarten Z-Ringelementen (100d, 100p) miteinander verbindet, und wobei nicht alle proximalen und distalen Täler des Stentmoduls miteinander verbunden sind.

4. Modularer Stent nach einem der vorherigen Ansprüche, bei welchem jeder der ringförmigen geschlossenzelligen Ringe (80c) durch lineare Streben gebildet ist.

5. Modularer Stent nach einem der Ansprüche 1 bis 3, bei welchem jeder der ringförmigen geschlossenzelligen Ringe (80c) durch nichtlinear Streben gebildet ist.

6. Modularer Stent nach einem der Ansprüche 1 bis 4, bei welchem jeder der ringförmigen geschlossenzelligen Ringe (80c) durch serpentinenförmige Streben gebildet ist.

7. Modularer Stent nach einem der vorherigen Ansprüche, bei welchem jeder der ringförmigen Z-Ringe durch im Wesentlichen lineare Streben gebildet ist.

8. Modularer Stent nach einem der Ansprüche 1 bis 7, bei welchem jeder der ringförmigen Ringe durch serpentinenförmige Streben gebildet ist.

9. Modularer Stent nach einem der Ansprüche 1 bis 6, bei welchem jeder der ringförmigen Z-Ringe durch im Wesentlichen nichtlineare Streben gebildet ist.

10. Modularer Stent nach einem der Ansprüche 1 bis 7, bei welchem jeder der ringförmigen Z-Ringe integrale glatte sinusförmige Wellen umfasst.

11. Modularer Stent nach einem der vorherigen Ansprüche, bei welchem jedes geschlossenzellige Segment (78c) zumindest zwei ringförmige geschlossenzellige Ringe (80c) sowie Verbinder, die Paare von in Längsrichtung benachbarten ringförmigen geschlossenzelligen Ringen (80c) miteinander verbinden, umfasst.

12. Modularer Stent nach einem der vorherigen Ansprüche, bei welchem jedes Z-Segment (82z, 84z) zumindest zwei ringförmige Z-Ringe sowie Verbinder, die Paare von in Längsrichtung benachbarten ringförmigen Z-Ringe miteinander verbinden, umfasst.

13. Stent nach Anspruch 12, bei welchem der Verbinder jedes Paar von axial benachbarten Z-Ringen bei in Umfangsrichtung mit Abstand versehenen Orten miteinander verbindet, und wobei nicht alle Orte gleichmäßig mit Abstand versehen sind.

14. Modularer Stent nach einem der Ansprüche 11 bis 13, bei welchem die Verbinder lineare Verbinder umfassen.

15. Modularer Stent nach einem der Ansprüche 11 bis 13, bei welchem die Verbinder serpentinenförmige Verbinder umfassen.

16. Modularer Stent nach einem der Ansprüche 11 bis 13, bei welchem die Verbinder gemeinsame Streben der zwei miteinander verbundenen ringförmigen geschlossenzelligen Ringe (80c) umfassen.

17. Modularer Stent nach einem der vorherigen Ansprüche, bei welchem jedes geschlossenzellige Segment zumindest einen geschlossenzelligen Ring mit 4 bis 16 geschlossenzelligen Elementen umfasst, die 4 bis 16 proximale und distale Spitzen und Täler definieren.

18. Modularer Stent nach einem der vorherigen Ansprüche, bei welchem jedes Z-Segment zumindest einen Z-Ring umfasst, der 4 bis 16 distale und proximale Spitzen und Täler definiert.

19. Modularer Stent nach einem der vorherigen Ansprüche, bei welchem der Stent aus fenestriertem Draht oder poröser metallischer Legierung (418) gebildet ist.

20. Modularer Stent nach einem der vorherigen Ansprüche, bei welchem der Stent eine Fähigkeit zum Eluieren von Medikamenten aufweist, aufgebracht direkt auf seine Oberfläche oder mittels eines Polymers oder einer anderen Beschichtung.

21. Modularer Stent nach einem der vorherigen Ansprüche, bei welchem der Stent aus einer Formgedächtnislegierung gebildet ist.

22. Modularer Stent nach Anspruch 21, bei welchem die Formgedächtnislegierung superelastisches Nitinol umfasst.

23. Modularer Stent nach Anspruch 21, bei welchem der Stent aus röhrenförmigem Material gebildet ist.

24. Modularer Stent nach Anspruch 21, bei welchem der Stent aus einem Drahtmaterial gebildet ist.

25. Modularer Stent nach einem der vorherigen Ansprüche, bei welchem der Stent ein ballonexpandierbarer Stent ist.

26. Modularer Stent nach einem der vorherigen Ansprüche, aufweisend einen äußersten ringförmigen Ring bei jedem Längsende des Stents, und wobei zumindest ein strahlenundurchlässiges Markierungsmittel auf zumindest einen der äußersten ringförmigen
Ringe des Stents aufgebracht ist.

27. Modularer Stent nach Anspruch 1, bei welchem jedes geschlossenzellige Segment 1 bis 4 geschlossenzellige Ringe umfasst, wobei jeder geschlossenzellige Ring 4 bis 16 geschlossenzellige Elemente und 4 bis 16 distale und proximale Spitzen aufweist; und
jedes Z-Segment 1 bis 8 Z-Ringe umfasst, wobei jeder Z-Ring 4 bis 16 distale und proximale Spitzen aufweist.

## Revendications

1. Stent modulaire ayant une configuration non déployée et une configuration tubulaire déployée, ledit stent comprenant au moins deux modules de stent (72i, 74d ; 502i, 504d), **caractérisé en ce que** :
au moins un module de stent (72i ; 502i) inclut:
un segment intermédiaire (78c ; 508c) consistant en un segment à cellules fermées (78c ; 508c) ;
une paire de segments d'extrémité (82z, 84z ; 512z, 516z) raccordés aux extrémités longitudinales respectives dudit segment intermédiaire (78c ; 508c),
chaque segment d'extrémité (82z, 84z ; 512z, 516z) consistant en un segment en Z (82z, 84z ; 512z,516z),
chaque segment en Z (82z, 84z ; 512z, 516z) consistant uniquement en au moins une bague en Z annulaire (85z, 87z ; 514z, 518z) formée d'entretoises définissant un élément allongé incluant une pluralité de parties en forme d'onde ayant une pluralité de pics et de creux proximaux et distaux ;
chaque segment à cellules fermées (78c, 508c), consistant uniquement en au moins une bague à cellules fermées annulaire (80c ; 510c) formée d'entretoises définissant une pluralité d'éléments à cellules fermées, ayant une pluralité de pics et de creux proximaux et distaux ; et
au moins un deuxième module de stent (74d ; 504d) inclut :
un segment intermédiaire (86z ; 520c) consistant en l'un d'un segment à cellules fermées (520c) ou d'un segment en Z (86z) ;
une paire de segments d'extrémité (89c, 90c ; 524z, 528z) raccordés aux extrémités longitudinales respectives dudit segment intermédiaire (86z ; 520c),
chaque segment d'extrémité (89c, 90c ; 524z, 528z) consistant en l'autre dudit segment à cellules fermées (89c, 90c) ou dudit segment en Z (524z, 528z) ;
chaque segment à cellules fermées (89c, 90c ; 520c) consistant uniquement en au moins une bague à cellules fermées annulaire (92c ; 522c) formée d'entretoises définissant une pluralité d'éléments à cellules fermées, ayant une pluralité de pics et de creux proximaux et distaux ;
chaque segment en Z (86z ; 524z, 528z) consistant uniquement en au moins une bague en Z annulaire (8z ; 526z, 530z) formée d'entretoises définissant un élément allongé incluant une pluralité de parties en forme d'onde, ayant une pluralité de pics et de creux proximaux et distaux.

2. Stent modulaire selon la revendication 1, dans lequel chaque bague à cellules fermées (80c) est formée d'une paire de bagues en Z opposées interconnectées étroitement aux paires de pics opposés ou aux paires de creux opposés ; ou aux paires de pics et de creux opposés.

3. Stent selon la revendication 1, dans lequel une pluralité d'éléments de raccordement espacés de façon circonférentielle (T) interconnectent chaque paire d'éléments de bague en Z adjacents axialement (100d, 100p), et dans lequel les creux proximaux et distaux dudit module de stent ne sont pas tous interconnectés.

4. Stent modulaire selon l'une quelconque des revendications précédentes, dans lequel chacune desdites bagues à cellules fermées annulaires (80c) est formée d'entretoises linéaires.

5. Stent modulaire selon l'une quelconque des revendications 1 à 3, dans lequel chacune desdites bagues à cellules fermées annulaires (80c) est formée d'entretoises non linéaires.

6. Stent modulaire selon l'une quelconque des revendications 1 à 5, dans lequel chacune desdites bagues à cellules fermées annulaires (80c) est formée d'entretoises en forme de serpentin.

7. Stent modulaire selon l'une quelconque des revendications précédentes, dans lequel chacune desdites bagues en Z annulaires est formée d'entretoises sensiblement linéaires.

8. Stent modulaire selon l'une quelconque des revendications 1 à 7, dans lequel chacune desdites bagues en Z annulaires est formée d'entretoises en forme de serpentin.

9. Stent modulaire selon l'une quelconque des revendications 1 à 6, dans lequel chacune desdites bagues en Z annulaires est formée d'entretoises sensiblement non linéaires.

10. Stent modulaire selon l'une quelconque des revendications 1 à 7, dans lequel chacune desdites bagues en Z annulaires comprend des ondes sinusoïdales régulières intégrales.

11. Stent modulaire selon l'une quelconque des revendications précédentes, dans lequel chaque segment à cellules fermées (78c) comprend au moins deux bagues à cellules fermées annulaires (80c), et des interconnecteurs interconnectant les paires de bagues à cellules fermées annulaires adjacentes de façon longitudinale (80c).

12. Stent modulaire selon l'une quelconque des revendications précédentes, dans lequel chaque segment en Z (82z, 84z) comprend au moins deux bagues en Z annulaires et des interconnecteurs interconnectant les paires de bagues en Z annulaires adjacentes longitudinalement.

13. Stent selon la revendication 12, dans lequel ledit interconnecteur interconnecte chaque paire de bagues en Z adjacentes axialement, en des emplacements espacés de façon circonférentielle, et dans lequel les emplacements ne sont pas tous espacés uniformément.

14. Stent modulaire selon l'une quelconque des revendications 11 à 13, dans lequel lesdits interconnecteurs comprennent des interconnecteurs linéaires.

15. Stent modulaire selon l'une quelconque des revendications 11 à 13, dans lequel lesdits interconnecteurs comprennent des interconnecteurs en forme de serpentin.

16. Stent modulaire selon l'une quelconque des revendications 11 à 13, dans lequel lesdits interconnecteurs comprennent des entretoises partagées desdites deux bagues à cellules fermées annulaires interconnectées (80c).

17. Stent modulaire selon l'une quelconque des revendications précédentes, dans lequel chaque segment à cellules fermées inclut au moins une bague à cellules fermées ayant 4 à 16 éléments à cellules fermées définissant 4 à 16 pics et creux proximaux et distaux.

18. Stent modulaire selon l'une quelconque des revendications précédentes, dans lequel chaque segment en Z inclut au moins une bague en Z définissant 4 à 16 pics et creux distaux et proximaux.

19. Stent modulaire selon l'une quelconque des revendications précédentes, dans lequel ledit stent est formé d'un alliage métallique poreux ou d'un fil fenêtré (418).

20. Stent modulaire selon l'une quelconque des revendications précédentes, dans lequel ledit stent a une capacité d'élution des médicaments, appliqués directement sur sa surface ou au moyen d'un polymère ou de tout autre revêtement.

21. Stent modulaire selon l'une quelconque des revendications précédentes, dans lequel ledit stent est formé d'un alliage à mémoire de forme.

22. Stent modulaire selon la revendication 21, dans lequel ledit alliage à mémoire de forme comprend du nitinol superélastique.

23. Stent modulaire selon la revendication 21, dans lequel ledit stent est formé d'un matériau tubulaire.

24. Stent modulaire selon la revendication 21, dans lequel ledit stent est formé d'un matériau de fil.

25. Stent modulaire selon l'une quelconque des revendications précédentes, dans lequel ledit stent est un stent déployable à ballonnet.

26. Stent modulaire selon l'une quelconque des revendications précédentes, ayant une bague annulaire la plus externe au niveau de chaque extrémité longitudinale du stent, et dans lequel au moins un marqueur radioopaque est appliqué sur au moins l'une desdites bagues annulaires les plus externes du stent.

27. Stent modulaire selon la revendication 1, dans lequel chaque segment à cellules fermées inclut 1 à 4 bagues à cellules fermées, chaque bague à cellules fermées ayant 4 à 16 éléments à cellules fermées et 4 à 16 pics distaux et proximaux ; et
chaque segment en Z inclut 1 à 8 bagues en Z, chaque bague en Z ayant 4 à 16 pics distaux et proximaux.
